# EUROPEAN PATENT APPLICATION

(11) **EP 4 682 269 A1**
(43) Date of publication of application: **21.01.2026**
(21) Application number: 24767249.6
(22) Date of filing: 08.03.2024
(51) Int. Cl.: C12Q 1/6855, C12N 15/10, C12Q 1/686, C12Q 1/6869

(54) **METHOD FOR AMPLIFYING COMPLIMENTARY DNA STRAND**

(30) Priority: 09.03.2023 JP 2023036275
(71) Applicant: Immunogeneteqs, Inc., Kashiwa-shi, Chiba 277-0882 (JP)
(72) Inventor: SHICHINO, Shigeyuki, Chiba 278-0022 (JP)
(74) Representative: Mewburn Ellis LLP
(86) International application number: PCT/JP2024/008998
(87) International publication number: WO 2024/185884

(57) **Abstract**

Disclosed is a cDNA amplification method capable of comprehensively amplifying mRNA with a high efficiency and a low bias even from a very small amount of cell sample. The method of amplifying a complementary DNA strand of the present invention comprises steps of capturing a target RNA and synthesizing a complementary DNA strand using a reverse transcriptase having terminal transferase activity in a liquid-phase system or a solid-phase system such that bases such as CCC are added to an end of the complementary DNA strand, and thereafter carrying out homopolymer tailing by the TdT reaction in the presence of a chain-terminating nucleotide triphosphate. By combination with a template-switching reaction, the method enables amplification of the complementary DNA strand with a higher efficiency and a lower bias.

## Description

### TECHNICAL FIELD

The present invention relates to a method of amplifying a complementary DNA strand, which method enables highly sensitive amplification of cDNA from a minute amount of mRNA.

### BACKGROUND ART

Recently, comprehensive gene expression analysis has been carried out using minute amounts of samples such as single individual cells. Such a technique has enabled single-cell RNA-seq analysis (scRNA-seq analysis) in which properties of several thousand to several hundred thousand individual cells are comprehensively understood, thereby enabling identification of the presence or absence of diversity within a cell population, identification of novel cell subpopulations, and identification of special cell populations related to pathological conditions. Furthermore, for example, an international project aiming at preparation of a single-cell-level atlas in humans has started in 2016 (Non-Patent Document 1). The technique is thus serving as a driving force for understanding of biology with a fine resolution that has never been achieved before.

scRNA-seq analysis is especially useful for the elucidation of complex immune mechanisms in pathological conditions. Representative examples of minute amounts of samples therefor include tumor-infiltrating T cells contained in biopsy specimens of primary lesions and metastatic lesions collected for the purpose of diagnosis in cancer patients, and B cells that specifically react with viruses and with vaccines against such viruses. From the viewpoint of the fact that cytotoxic T lymphocytes (CTLs) are essential in immunotherapies using anti-PD-1 antibody or the like, and that they are actually CD8⁺ T cells, methods of measuring the extent to which CD8⁺ T cells capable of specific recognition of cancer antigens are induced are attracting attention (Non-Patent Document 2). Further, in the SARS-CoV2 pandemic from 2019, and in the development of vaccines effective against it, attention has been focused on investigating the properties of B cells with a high capacity to produce an antibody having strong neutralizing activity against the virus, and investigating the properties of CD4/CD8 T cells that react in an antigen-specific manner. In one of such measurement methods, the sequences of T cell receptors (TCRs) on T cells and the sequences of B cell receptors (BCRs) on B cells are analyzed to determine the TCR/BCR repertoire (the clone types and the relative clonal frequency), and at the same time, scRNA-seq analysis is carried out, to discuss the responses of antigen-specific clones and differences in the properties of those cells (scTCR/BCR/RNA-seq). It has actually been reported that there is a correlation between the fluctuation of the TCR repertoire and the therapeutic effect of an anti-CD4 antibody against a cancer, and that a plurality of times of vaccination against SARS-CoV2 leads to changes in the types of T cells (Non-Patent Documents 3 and 4). Ideally, in order to realize equivalent discussion on the gene expression of such cell types responsible for the immune response, mRNA should be obtained in equivalent amounts from those cells. However, the amounts of mRNA contained in T/B cells are much smaller than those of epithelial cells, tumor cells, macrophages, and the like. Therefore, to enable high-resolution scTCR/BCR/RNA-seq analysis of such cells, it is becoming increasingly important to develop a method for highly sensitive amplification of mRNA from individual cells derived from a specimen.

As methods of amplifying cDNA from a minute amount of sample, a variety of techniques are known. cDNA amplification methods can be classified based on whether or not mRNA capture and cDNA synthesis are carried out on a solid phase. Examples of the solid-phase system, where mRNA is captured in a microwell or a microdroplet by an mRNA-capture oligo immobilized on a solid-phase bead to carry out cDNA synthesis, include systems utilizing a plate having microwells such as the BD Rhapsody system from Beckton Dickinson (Patent Document 3), the Seq-well system (Non-Patent Document 12), and the Nx1-seq system (Patent Document 5, Non-Patent Document 14); and systems in which mRNA is captured in a microdroplet such as the Drop-seq method (Non-Patent Document 13) and the Nadia system (Dolomite Bio). Examples of the liquid-phase system, where mRNA is captured in a microwell or a microdroplet by a free mRNA-capture oligo not bound to a solid phase to carry out cDNA synthesis, include the 10X Chromium system (10X Genomics), in which a single cell and a hydrogel bead containing mRNA-capture oligos encapsulated therein are encapsulated in a microdroplet, and melting of the hydrogel and lysis of the cell are allowed to occur in the microdroplet to capture mRNA with the released mRNA-capture oligos (Patent Document 4); and Smart-Seq2, in which amplification is performed after dispensing individual cells or a minute amount of mRNA on a 96-well plate (Non-Patent Document 15). Another example of the liquid-phase analysis techniques is combinatorial indexing, such as sci-RNA-seq3, in which fixed cells are suspended in a solution containing a cell membrane permeabilization reagent and mRNA-capture oligos and mRNA is captured on the mRNA-capture oligos in the fixed cells.

Focusing on reaction modes such as that of the second-strand synthesis reaction, methods of amplifying cDNA from a minute amount of sample can also be classified into the following two types. The first is the template switching (TS) method (Patent Document 1, Non-Patent Document 5, Non-Patent Document 6). In this method, a primer of several ten base pairs called template-switching oligo, which has a base sequence such as [guanosine]-[guanosine]-[guanosine] at the 3'-end, is added during the cDNA synthesis from mRNA, thereby appending a desired base sequence at the cDNA terminus and synthesizing the second strand. The second is the TdT method (Non-Patent Documents 7 to 9). In this method, a polynucleotide sequence composed of identical bases is added to the terminus of the synthesized cDNA using terminal deoxynucleotidyl transferase (TdT), and the second strand is synthesized using a primer that has the sequence complementary to this polynucleotide at its 3'-end and a known adapter sequence at its 5'-end.

The TS method has the advantages that the loss of cDNA associated with purification is low since the method requires only a small number of reaction steps, and that full-length cDNA is readily generated due to its nature that the TS reaction occurs predominantly on mRNA having the 5' cap structure. On the other hand, the method has the disadvantage that the efficiency of the TS reaction is highly dependent on the terminal structure of the mRNA. For example, the efficiency decreases depending on the terminal base in the order of G>A>C>U, and, while relatively high efficiency (within the range of about 20% to 60%) is achieved for full-length cDNA having a cap structure at the 5'-end, the efficiency is low (within the range of several percent to 40%) when mRNA without the cap structure such as non-full-length mRNA, immature mRNA, or partially degraded mRNA is involved. Such uneven efficiency leads to an amplification bias (Non-Patent Document 6). Another disadvantage is that the polynucleotide addition efficiency of the TS method is lower than that of the TdT method (92 to 95%, Non-Patent Document 10).

On the other hand, although the TdT method achieves higher efficiency than the TS method, the TdT method has the disadvantages that control of the TdT reaction is difficult, that a lot of steps are required, and that, in cases where a single-strand DNA is used as a template, the efficiency may largely decrease when a higher order structure is formed at its terminus to form a recessed end (Non-Patent Document 10), so that long-chain full-length cDNAs, which more easily form a higher order structure, are less likely to be synthesized compared to the TS reaction.

The TAS-Seq method, which has previously been developed by the present inventors (Patent Document 2, Non-Patent Document 11), is a technique that solves the difficulty in controlling the TdT reaction in the TdT method. The TAS-Seq method is a solid-phase cDNA amplification method in which a target nucleic acid is captured on a solid-phase for the synthesis of cDNA. In this method, a predetermined amount of chain-terminating nucleotide triphosphate is added to a TdT reaction system, and the TdT reaction is stochastically controlled such that the nucleotide homopolymer addition reaction by the TdT reaction occurs within a predetermined range even under diverse conditions. As a result, the TAS-Seq method is excellent in the detection sensitivity for expressed genes and the quantification of the cell composition, by which intercellular communications, which is difficult to detect by conventional techniques, can also be detect more robustly.

### PRIOR ART DOCUMENTS

### PATENT DOCUMENTS

Patent Document 1: WO 2015/027135 A1
Patent Document 2: WO 2021/006353 A1
Patent Document 3: US 2018/0258500 A1
Patent Document 4: WO 2015/200893 A2
Patent Document 5: WO 2015/166768 A1

### NON-PATENT DOCUMENTS

Non-Patent Document 1: Regev A., et al. The Human Cell Atlas. eLife 2017; e27041.
Non-Patent Document 2: Zaretsky JM, Garcia-Diaz A, Shin DS, et al. Mutations Associated with Acquired Resistance to PD-1 Blockade in Melanoma. N Engl J Med 2016; 375:819-2.
Non-Patent Document 3: Aoki H., et al. TCR Repertoire Analysis Reveals Mobilization of Novel CD8+ T Cell Clones Into the Cancer-Immunity Cycle Following Anti-CD4 Antibody Administration. Front Immunol. 2019 Jan; 9(3185).
Non-Patent Document 4: Aoki H., et al.. T cell responses induced by SARS-CoV-2 mRNA vaccination are associated with clonal replacement bioRxiv. 2022 Aug.
Non-Patent Document 5: Picelli S., et al. Full-length RNA-seq from single cells using Smart-seq2. Nat Protoc. 2014 Jan; 9(1):171-81.
Non-Patent Document 6: Wulf MG., et al. Non-templated addition and template switching by Moloney murine leukemia virus (MMLV)-based reverse transcriptases co-occur and compete with each other. J Biol. Chem. 2019 Nov; 294(48):18220-18231.
Non-Patent Document 7: Matsunaga H, Goto M, Arikawa K, et al. A highly sensitive and accurate gene expression analysis by sequencing ("bead-seq") for a single cell. Anal Biochem 2015; 471:9-16.
Non-Patent Document 8: Sasagawa Y., Nikaido I., Hayashi T., Danno H., Uno, K. D., Imai T., Ueda H. R., Quartz-Seq: a highly reproducible and sensitive single-cell RNA sequencing method, reveals non-genetic gene-expression heterogeneity. Genome Biol 2013 Apr; 14(4)
Non-Patent Document 9: Tang F, Barbacioru C, Wang Y, et al. mRNA-Seq whole-transcriptome analysis of a single cell. Nat Methods 2009, 6(5):377-382
Non-Patent Document 10: Deng G, Wu R. An improved procedure for utilizing terminal transferase to add homopolymers to the 3' termini of DNA. Nucleic Acid Res 1981, 9(16):4173-4188.
Non-Patent Document 11: Shichino S, Ueha S, Hashimoto S, et al. TAS-Seq is a robust and sensitive amplification method for bead-based scRNA-seq. Commun Biol. 5:602-602, 2022.
Non-Patent Document 12: Gierahn TM et al. Seq-Well: portable, low-cost RNA sequencing of single cells at high throughput. Nat Methods 2017; 14(4):395-398.
Non-Patent Document 13: Macosko EZ et al. Highly Parallel Genome-wide Expression Profiling of Individual Cells Using Nanoliter Droplets. Cell 2015; 161(5):1202-1214.
Non-Patent Document 14: Hashimoto et al. Scientific Reports 2017 Oct 27; 7(1):14225. doi: 10.1038/s41598-017-14676-3.
Non-Patent Document 15: Picelli S., et al. Full-length RNA-seq from single cells using Smart-seq2. Nat Protoc. 2014 Jan; 9(1):171-81.

### SUMMARY OF THE INVENTION

### PROBLEMS TO BE SOLVED BY THE INVENTION

Development of a method that enables highly sensitive, low-biased amplification of mRNA from single individual cells derived from various specimens is an important challenge from the viewpoint of, for example, construction of a more advanced and more precise single-cell atlas, capturing of tumor-reactive T cells, and capturing of B cells having high vaccine responsiveness. However, the existing TS and TdT methods have their own advantages and disadvantages as described above. A highly sensitive cDNA amplification method that retains all of the advantages does not yet exist. The TAS-Seq method, which was previously developed by the present inventors, also has a problem due to the nature of the method. That is, since a predetermined amount of terminator nucleotides is added in this method, cDNA molecules to which a polynucleotide with a length sufficient to form a stable complementary strand with a primer used in the second-strand synthesis is not appended are inevitably produced at a certain amount. Since the TAS-Seq method is a type of the TdT method, it is similar to the common TS method in terms of the facts that more steps are required than the TS method, and that long-chain full-length cDNA molecules are less likely to be synthesized compared to the TS reaction. Further, since the known TAS-Seq method is a solid-phase cDNA amplification method, its application to a liquid-phase system has not been known.

An object of the present invention is to provide a cDNA amplification method capable of highly efficiently and comprehensively amplifying mRNA with a low bias even from a very small amount of cell sample.

### MEANS FOR SOLVING THE PROBLEMS

When the TS method and the TdT method are combined, it is expected that the increase in the cDNA yield because of the smaller number of reaction steps, which is an advantage of the TS method, cannot be achieved, and that the high reaction efficiency, which is an advantage of the TdT method, cannot be achieved since formation of a protruding end, which is a preferred end structure for the TdT reaction, may be prevented due to exposure of the 3'-end of cDNA during the template-switching reaction in the TS method. Further, in the liquid-phase TS method, in order to avoid loss of the product during the purification process, removal of unreacted target RNA-capture oligos (i.e., target RNA-capture oligos that have not captured the target) is usually not performed after the cDNA synthesis (Non-Patent Document 15). That means, there is a common technical knowledge that, if a purification step is carried out for removing unreacted target RNA-capture oligos after the cDNA synthesis in the liquid-phase TS method, the cDNA product decreases, resulting in a decrease in the final cDNA amplification efficiency. On the other hand, if the TdT method is applied as it is to the product obtained after applying the TS method without performing the step of removal of unreacted target RNA-capture oligos according to the above-mentioned common technical knowledge, it is expected that the unreacted target RNA-capture oligos and unreacted template-switching oligos, which are present in much larger amounts than the cDNA product, are preferentially amplified by the TdT method, so that the cDNA amplification efficiency largely decreases. Consequently, no method combining the TS and TdT methods has been reported. Furthermore, there is no basis to expect any benefit great enough to compensate for canceling out the respective advantages of each method.

Nevertheless, also in the liquid-phase system, the present inventors attempted a step of removing unreacted target RNA-capture oligos and unreacted template-switching oligos from a product that had been subjected to the TS reaction, and the TdT method was further applied thereafter. As a result of this study, it was surprisingly discovered that the cDNA yield could be largely increased compared to cases where each reaction was carried out alone, that the amount of long-chain cDNA molecules synthesized increased compared to cases where the TdT reaction was carried out alone, and that the amount of short-chain cDNA molecules synthesized increased compared to cases where the TS reaction was carried out alone.

Furthermore, the present inventors discovered that, in cases where the step of TAS-Seq (TdT method) is carried out using a reverse transcriptase having terminal transferase activity without combining the TS reaction, the ratio of cDNA molecules whose homopolymer portion (especially, poly(C)) is too short can be decreased due to addition of bases such as CCC to the cDNA terminus, so that the TAS-Seq method can be efficiently carried out even in a liquid-phase system that does not use a solid phase.

The present invention, completed by the above intensive study, is a method of amplifying a complementary DNA strand, the method comprising steps of capturing a target RNA and synthesizing a complementary DNA strand using a reverse transcriptase having terminal transferase activity in a liquid-phase system or a solid-phase system to add bases such as CCC to the terminus of the complementary DNA strand, and then carrying out homopolymer tailing by the TdT reaction in the presence of a chain-terminating nucleotide triphosphate. When a TS reaction using a template-switching oligo is further combined with this method, more efficient and lower-biased amplification of the complementary DNA strand can be achieved. The present invention includes the following modes.
[1] A method of amplifying a complementary DNA strand, the method comprising:
   a target RNA-capturing step of capturing a target RNA with a target RNA-capture oligo comprising a target capture portion;
   a complementary-strand synthesis step of carrying out reverse transcription reaction using a reverse transcriptase having terminal transferase activity, to synthesize a complementary DNA strand whose sequence is complementary to the captured RNA, and to add an additional sequence consisting of several arbitrary bases to the 3'-end of at least part of the complementary DNA molecules;
   an unreacted-oligo removal step of removing a target RNA-capture oligo that has not captured the target RNA;
   an RNA degradation step of degrading RNA using a ribonuclease;
   a homopolymer addition step of carrying out a reaction using terminal deoxynucleotidyl transferase in the presence of dATP, dTTP, dCTP or dGTP and a chain-terminating nucleotide triphosphate, to add a nucleotide homopolymer to the 3'-end of the complementary DNA strand;
   a second-strand synthesis step of synthesizing a second strand for the complementary DNA strand using a second-strand-synthesizing primer which comprises a primer comprising a complementary homopolymer portion whose sequence is complementary to the nucleotide homopolymer, to generate a DNA double strand composed of the complementary DNA strand and the second strand; and
   a nucleic acid amplification step of carrying out nucleic acid amplification reaction using the DNA double strand as a template.
[2] The method according to [1], wherein: the target RNA-capture oligo comprises a first adapter portion on the 5'-side of the target capture portion; the second-strand-synthesizing primer comprises a long primer comprising a second adapter portion on the 5'-side of the complementary homopolymer portion; and in the nucleic acid amplification step, nucleic acid amplification reaction is carried out using a primer targeting the first adapter and a primer targeting the second adapter.
[3] The method according to [1], wherein: the second-strand-synthesizing primer comprises a long primer comprising a second adapter portion on the 5'-side of the complementary homopolymer portion; and, in the nucleic acid amplification step, nucleic acid amplification reaction is carried out using a primer targeting the second adapter and a primer targeting a desired region in the complementary DNA strand.
[4] The method according to [2] or [3], wherein the long primer comprises, between the second adapter portion and the complementary homopolymer portion, a molecular barcode portion consisting of a random sequence.
[5] The method according to any one of [2] to [4], wherein the chain length of the complementary homopolymer portion of the long primer is 6 to 15 bases.
[6] The method according to any one of [1] to [5], wherein: the target RNA-capture oligo is a free oligo not bound to a solid-phase carrier; and the unreacted-oligo removal step is carried out by nucleic acid size fractionation.
[7] The method according to [6], wherein the target RNA-capture oligo comprises, from the 5'-side to the 3'-side, a first adapter portion, a cell identification barcode portion, and a target capture portion.
[8] The method according to any one of [1] to [5], wherein: the target RNA-capture oligo is a solid-phased oligo bound to a solid-phase carrier; and the unreacted-oligo removal step is carried out by exonuclease treatment.
[9] The method according to [8], wherein: the solid-phase carrier is a bead; and the target RNA-capture oligo comprises, from the 5'-side to the 3'-side, a first adapter portion, a bead identification barcode portion, and a target capture portion.
[10] The method according to [8], wherein: the solid-phase carrier is a plate; and the target RNA-capture oligo is immobilized on a plurality of compartments on the plate, and comprises, from the 5'-side to the 3'-side, a first adapter portion, a compartment identification barcode portion, and a target capture portion.
[11] The method according to any one of [1] to [10], wherein: the target RNA-capture oligo comprises a target poly(A) RNA-capture oligo comprising a poly(T) portion as a target capture portion; and the target RNA comprises a poly(A) RNA.
[12] The method according to [11], wherein in the complementary-strand synthesis step, reverse transcription reaction is carried out in the presence of a template-switching oligo comprising: a sequence capable of hybridizing with the additional sequence, at its 3'-end; and a switching sequence on the 5'-side of the sequence capable of hybridizing with the additional sequence; to hybridize the template-switching oligo with the complementary DNA strand having the additional sequence added thereto, and to further add a sequence complementary to the switching sequence to the 3'-end of the complementary DNA strand.
[13] The method according to [12], wherein the template-switching oligo comprises an oligonucleotide comprising, at its 3'-end, GGG, GUG, or NGG (wherein G and U are bases of ribonucleotides, and N is a base of any ribonucleotide selected from A, U, G, and C; and wherein one or more nucleotide analogues are optionally included) as the sequence capable of hybridizing with the additional sequence, and the switching sequence comprises a second adapter portion.
[14] The method according to [13], wherein the switching sequence comprises a molecular barcode portion consisting of a random sequence on the 3'-side of the second adapter portion.
[15] The method according to any one of [12] to [14], wherein the target RNA-capture oligo is a free oligo not bound to a solid-phase carrier, and wherein the unreacted-oligo removal step comprises removal of a target RNA-capture oligo that has not captured the target RNA and a template-switching oligo that has not hybridized with the complementary DNA strand by nucleic acid size fractionation.
[16] The method according to any one of [11] to [15], wherein the second-strand-synthesizing primer comprises: a long primer comprising a second adapter portion on the 5'-side of the complementary homopolymer portion; and a short primer comprising no complementary homopolymer portion and comprising a second adapter portion at its 3'-side region.
[17] The method according to [16], wherein the long primer comprises, between the second adapter portion and the complementary homopolymer portion, a molecular barcode portion consisting of a random sequence.
[18] The method according to any one of [12] to [17], wherein in the complementary-strand synthesis step, reverse transcription reaction is carried out using a reverse transcriptase further having strand displacement activity in the presence of the template-switching oligo, to carry out second-strand synthesis for the complementary DNA strand with which the template-switching oligo has been hybridized.
[19] The method according to any one of [1] to [18], wherein the chain-terminating nucleotide triphosphate is ddNTP, a ddNTP derivative, 3'-dNTP, or 3'-deoxy-5-methyluridine-5'-triphosphate.
[20] The method according to [19], wherein the ddNTP derivative is ddNTP in which the 3'-position is modified with an atomic group comprising no OH group.
[21] The method according to [19], wherein the chain-terminating nucleotide triphosphate is ddNTP.
[22] The method according to any one of [1] to [21], wherein in the homopolymer addition step, dCTP and chain-terminating CTP are added to carry out poly(C) addition.
[23] The method according to any one of [1] to [22], wherein the RNA degradation step and the homopolymer addition step are carried out simultaneously.

### EFFECT OF THE INVENTION

According to the present invention, use of a reverse transcriptase having terminal transferase activity enables more efficient and low-biased comprehensive amplification of mRNA compared to a conventional TAS-Seq method (Patent Document 2, Non-Patent Document 11) even from a very small amount of sample, and a TAS-Seq method can be efficiently carried out even in a liquid-phase system that does not use a solid phase. When combined with the TS reaction, the yields of both long-chain cDNA molecules and short-chain cDNA molecules can be largely increased. In particular, according to the conventional technical knowledge, a liquid-phase TS method is expected to have the problem that the cDNA amplification efficiency decreases regardless of whether or not a purification step is performed to remove unreacted oligos after the cDNA synthesis. However, contrary to this expectation, high efficiency and low bias can be achieved, and the yields of both long-chain cDNA molecules and short-chain cDNA molecules can be largely increased by the present invention. According to the method of the present invention, for example, the accuracy of determination of therapeutic effects utilizing expression analysis or TCR analysis, and separation and identification of rare cells (such as cancer cells) by scRNA-seq analysis can be largely improved compared to conventional methods. Thus, the method can further contribute to, for example, the reduction of excessive consumption of medical resources.

### BRIEF DESCRIPTION OF THE DRAWINGS

[Fig. 1] A scheme illustrating the steps of a liquid-phase nonTS/TAS-Seq method using a reverse transcriptase having terminal transferase activity, which is one mode of the present invention.
[Fig. 2] A scheme illustrating the steps of a solid-phase TS/TAS-Seq method using a reverse transcriptase having terminal transferase activity but having no strand displacement activity, which is one mode of the present invention.
[Fig. 3] A scheme illustrating the steps of a solid-phase TS/TAS-Seq method using a reverse transcriptase having terminal transferase activity and strand displacement activity, which is one mode of the present invention.
[Fig. 4] A scheme illustrating the steps of a liquid-phase TS/TAS-Seq method using a reverse transcriptase having terminal transferase activity but having no strand displacement activity, which is one mode of the present invention.
[Fig. 5] A scheme illustrating the steps of a liquid-phase TS/TAS-Seq method using a reverse transcriptase having terminal transferase activity and strand displacement activity, which is one mode of the present invention.
[Fig. 6] Comparison of the yield of the total cDNA amplification product between a TdT method and a template-switch + TdT method in a solid-phase system (BD Rhapsody single-cell analysis system). The template-switch + TdT method shows a significantly higher total cDNA yield.
[Fig. 7] Comparison of the size distribution of the total cDNA amplification product between a TdT method and a template-switch + TdT method in a solid-phase system (BD Rhapsody single-cell analysis system). The template-switch + TdT method shows a higher yield of long-chain cDNA molecules.
[Fig. 8] Comparison of the yield and the size distribution of the total cDNA amplification product between a TdT method and a template-switch + TdT method in a liquid-phase system (10X Chromium single-cell analysis system). The template-switch + TdT method shows a higher cDNA yield, and a higher yield of long-chain cDNAs.
[Fig. 9] Comparison of the yield of the total cDNA amplification product between a template-switch method and a template-switch + TdT method in a liquid-phase system (Smart-seq2 method). The template-switch + TdT method shows a significantly higher cDNA yield.
[Fig. 10] Comparison of the size distribution of the total cDNA amplification product between a template-switch method and a template-switch + TdT method in a liquid-phase system (Smart-seq2 method). The template-switch + TdT method shows a higher proportion of short-chain cDNA molecules in the total cDNA.
[Fig. 11] Comparison of the number of detected genes and the number of reads in each cell according to single-cell RNA sequence analysis data, among a TdT method, a random priming method, and a template-switch + TdT method in a solid-phase system (BD Rhapsody single-cell analysis system). The template-switch + TdT method was able to detect the largest number of genes under conditions with a similar distribution of the number of reads per cell.
[Fig. 12] Comparison of the number of detected genes and the number of reads in each cell according to single-cell RNA sequence analysis data, between a template-switch method and a template-switch + TdT method in a liquid-phase system (10X Chromium single-cell analysis system). The template-switch + TdT method was able to detect a larger number of genes under conditions with a similar distribution of the number of reads per cell.
[Fig. 13] Results of single-cell clustering analysis of single-cell RNA sequence analysis data by a template-switch method and a template-switch + TdT method in a liquid-phase system (10X Chromium single-cell analysis system). The template-switch + TdT method has an improved separation capacity for cell populations.
[Fig. 14] Comparison of the number of detected genes as observed for each cell subset when the number of reads was changed, according to single-cell RNA sequence analysis data by a template-switch method and a template-switch + TdT method in a liquid-phase system (10X Chromium single-cell analysis system). The template-switch + TdT method was able to detect a larger number of genes for all cell populations.

### MODE FOR CARRYING OUT THE INVENTION

In the present invention, A, T, G, and C constituting the base sequence of a target RNA-capture oligo, a template-switching oligo, a primer, an adapter, a nucleotide homopolymer, or the like include not only deoxyribonucleotides constituting DNA (A, T, G, and C of DNA), but also ribonucleotides constituting RNA (A, T, G, and C of RNA), and further, their corresponding nucleotide analogues (including bridged nucleic acids such as LNA, ENA, and PNA; and modified bases such as Super T (5-hydroxybutynl-2'-deoxyuridine), Super G (8-aza-7-deazaguanosine), deoxyinosine, 5-methyl dC, deoxyuridine, 2,6-diaminopurine, 2-aminopurine, and 2-Amino-dATP). Thus, the target RNA-capture oligo (for example, any of the target capture portion, the first adapter portion, the barcode portion, and other portions), the template-switching oligo (any of the 3'-end portion capable of hybridizing with the additional sequence, the second adapter portion, the barcode portion, and other portions), the nucleotide homopolymer, the second-strand-synthesizing primer (for example, any of the complementary homopolymer portion, the second adapter portion, the barcode portion, and others), and each primer used in the nucleic acid amplification step may contain one or more (for example, about 1 to 15, about 1 to 12, or about 1 to several) monomers selected from ribonucleotides and nucleotide analogues.

In the present invention, "several" means a plural number of 2 to 9. "One to several" may be one to nine, one to eight, one to seven, one to six, one to five, one to four, one to three, or one to two.

In the present invention, a primer targeting a region X means a primer that specifically hybridizes with the region X or with the complementary strand thereof. Examples of the primer targeting a region X include primers comprising the same sequence as the region X or comprising the sequence complementary thereto. The term "primer that is set in a region X" has the same meaning, and means a primer that specifically hybridizes with the region X or with the complementary strand thereof. Examples of the primer that is set in a region X include primers comprising the same sequence as the region X or comprising the sequence complementary thereto.

In the present invention, the terms "complementary DNA strand" and "cDNA" mean a DNA strand consisting of a base sequence complementary to a target RNA, and are not limited to a DNA strand consisting of a sequence complementary to mRNA.

In the present invention, the term "oligonucleotide" or "oligo" means a single-stranded polynucleotide with a relatively short chain length. The chain length of the oligonucleotide is typically about several ten bases to 200 bases, for example, about 50 bases to 150 bases. However, the chain length is not limited thereto.

In the present invention, the term "terminal transferase activity" means the activity of terminal deoxynucleotidyl transferase to add nucleotides to the 3'-end of a nucleotide chain in a template-independent manner. The 3'-end of a single-stranded nucleotide, a 3'-protruding end of a double-stranded nucleotide, and the 3'-end of a blunt end of a double-stranded nucleotide are targets of nucleotide addition by the terminal transferase activity.

In the present invention, a target RNA encompasses a variety of RNA molecules including poly(A) RNA. Poly(A) RNA is RNA having a poly(A) tail at the 3'-end, and the most typical example thereof is mRNA. The cell from which the target RNA is derived is not limited, and examples of such a cell include various cells such as cells derived from mouse individuals, various cultured cells, cells derived from healthy humans, cells derived from cancer patients, and cells derived from patients with various diseases. The cells derived from cancer patients may be cells derived from cancer patients receiving cancer immunotherapy. The cells derived from healthy humans or patients may be cells collected from peripheral blood or a lesion (such as a tumor). In cases where the amplification method of the present invention is used in analysis methods such as transcriptome analysis, cells may be used as a sample, or cell nuclei isolated from various cells may be used as a sample.

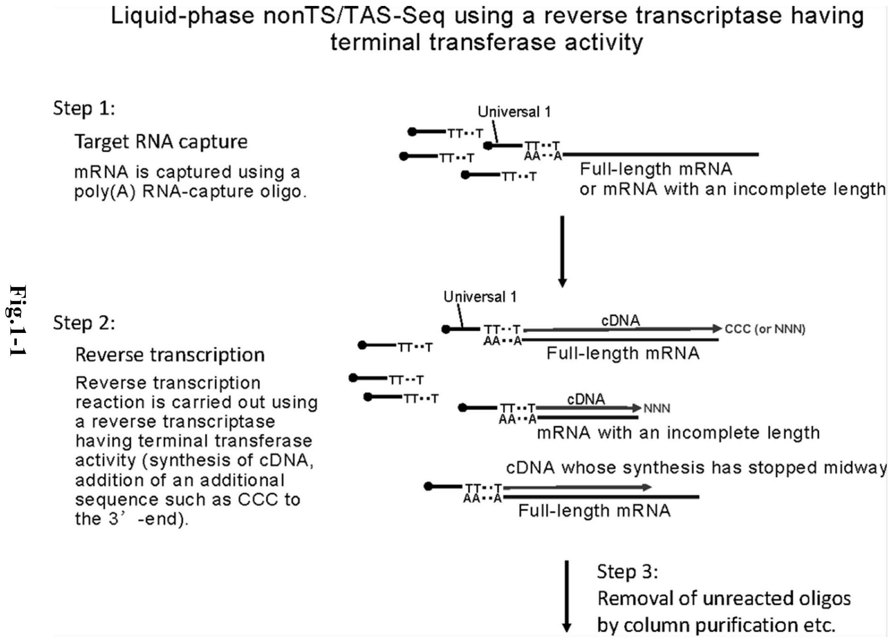

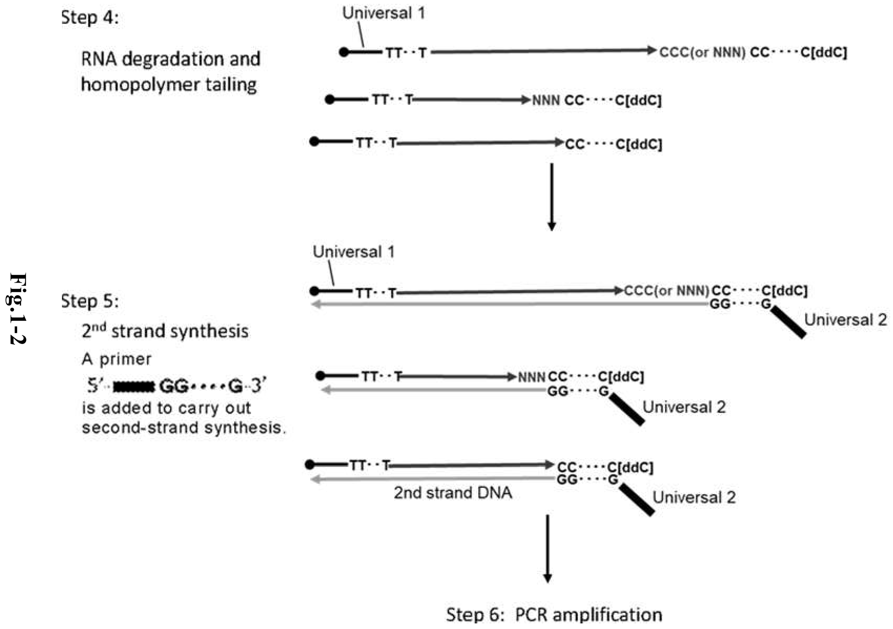

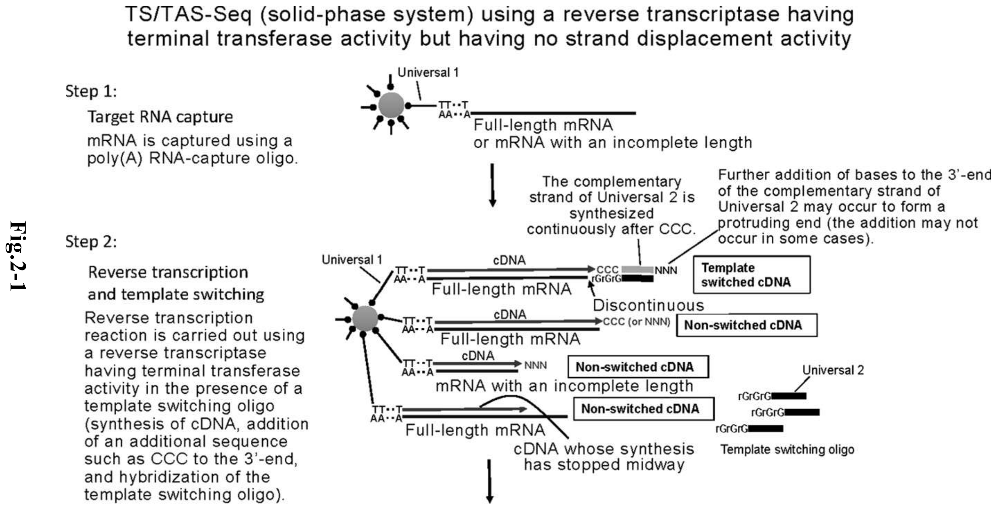

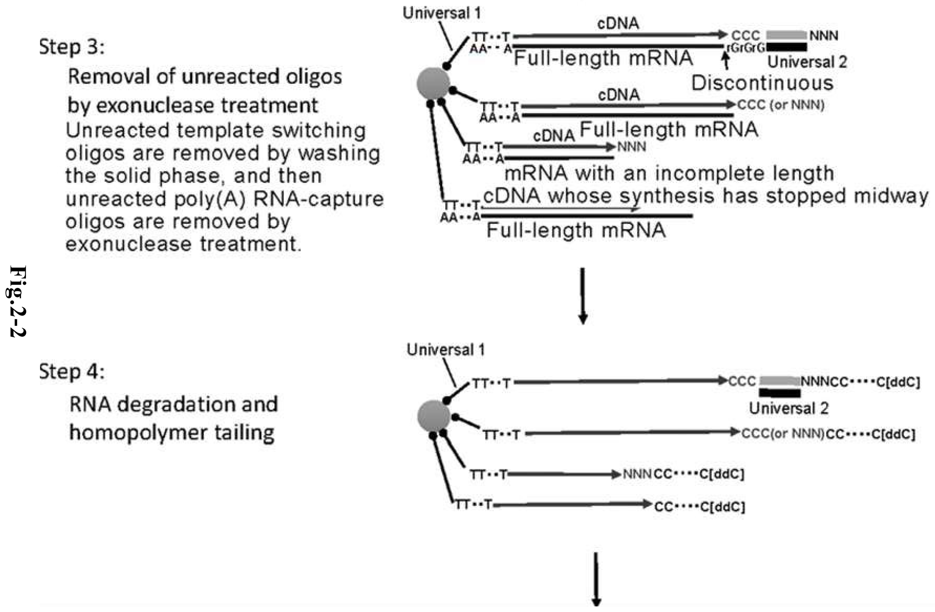

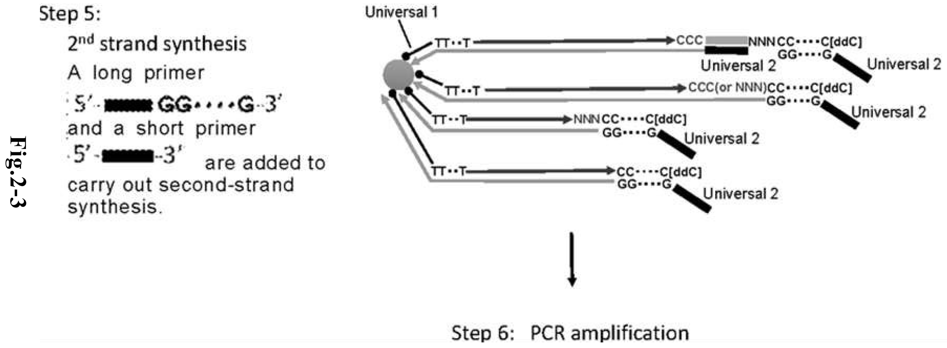

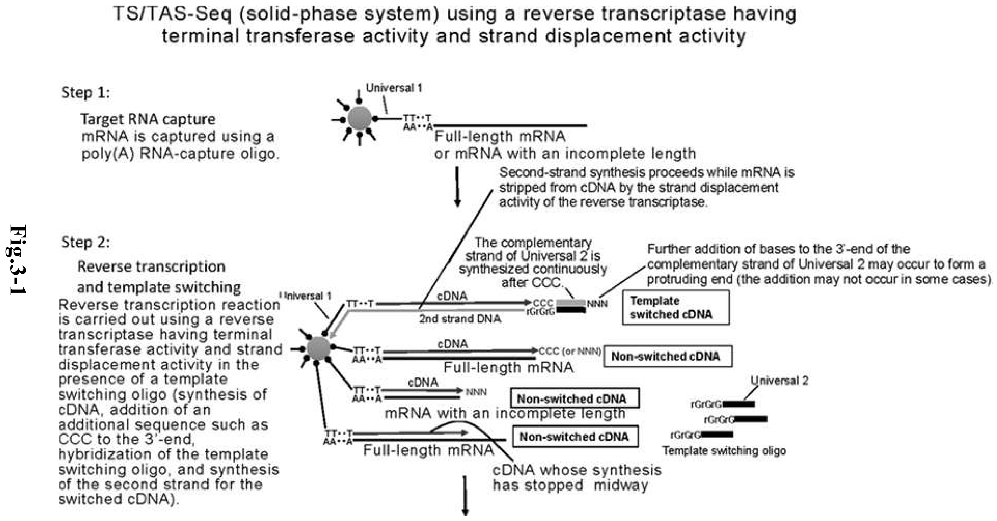

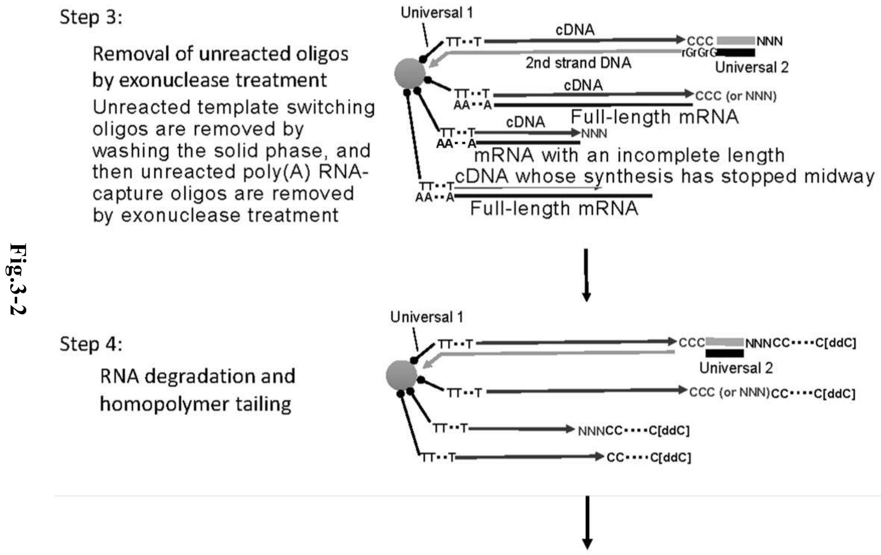

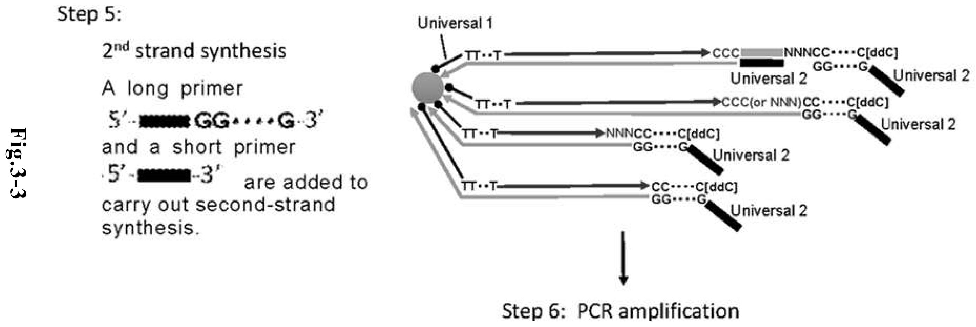

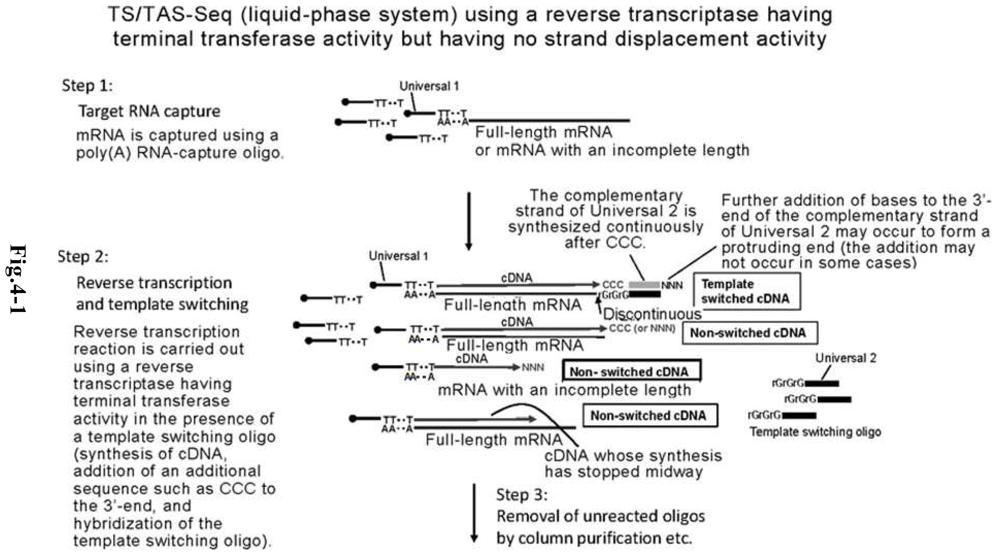

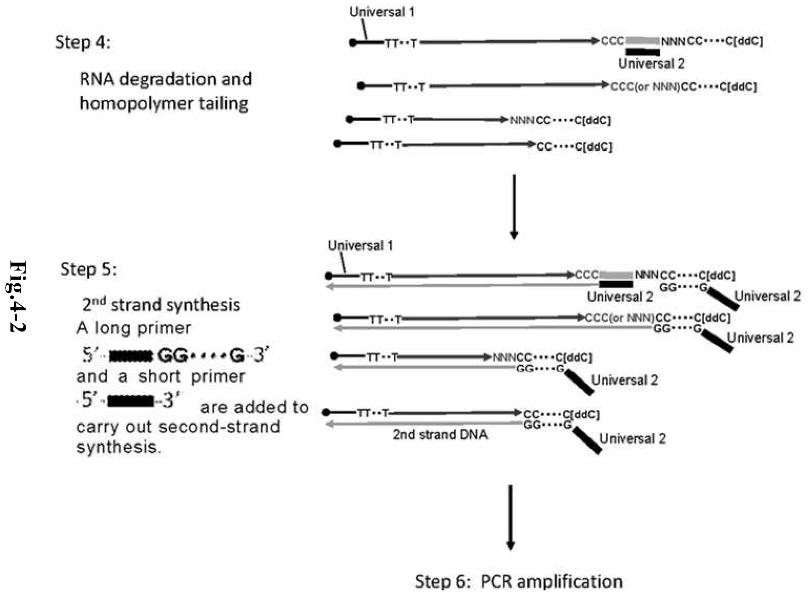

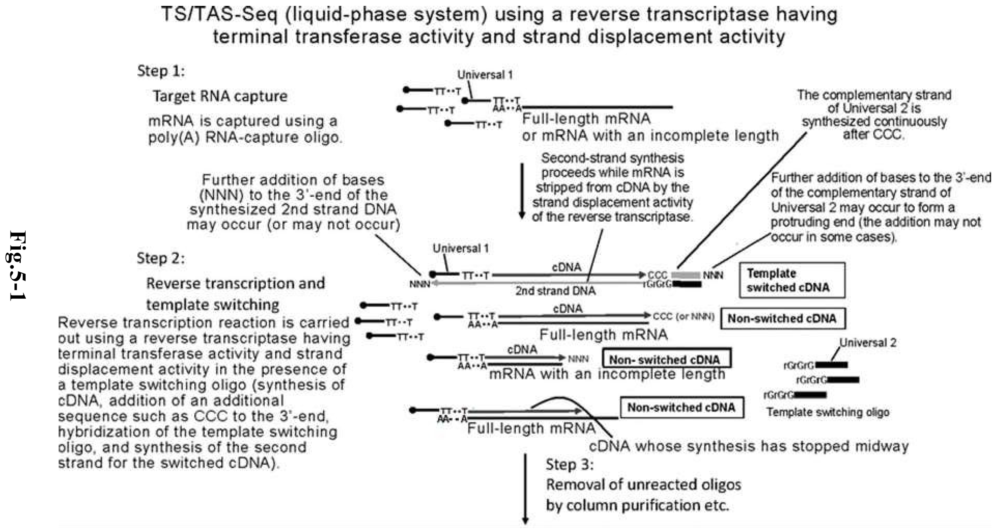

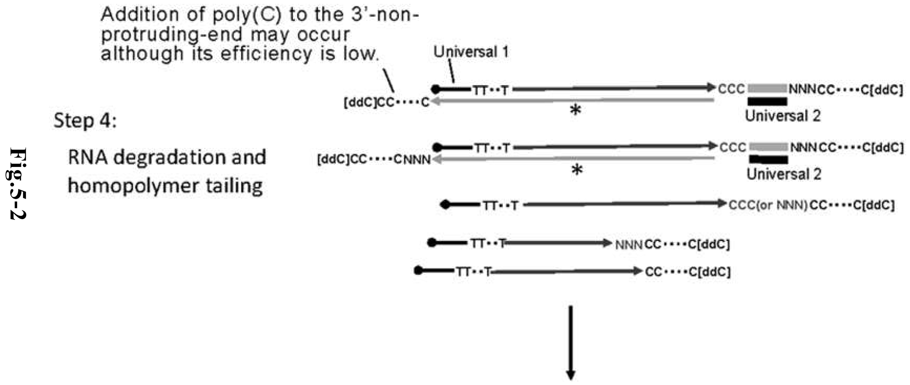

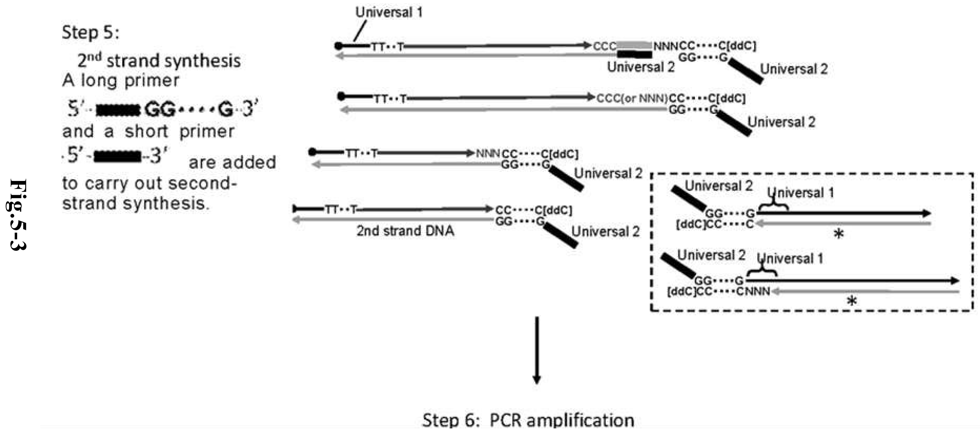

The method of amplifying a complementary DNA strand of the present invention comprises a target RNA-capturing step, a complementary-strand synthesis step, an unreacted-oligo removal step, an RNA degradation step, a homopolymer addition step, a second-strand synthesis step, and a nucleic acid amplification step. The amplification method of the present invention encompasses a mode in which, in the complementary-strand synthesis step, reverse transcription reaction is carried out in the presence of a template-switching oligo to perform template-switching (TS) reaction, and a mode in which the reverse transcription reaction is carried out in the absence of the template-switching oligo. For convenience, the former mode is referred to as the TS/TAS-Seq method, and the latter mode is referred to as the nonTS/TAS-Seq method. The TS/TAS-Seq method is more preferred as a mode of the amplification method of the present invention. The nonTS/TAS-Seq method is preferred in cases where the TS reaction causes production of an extremely undesirable product (for example, a strand invasion product due to the template-switching oligo, a cDNA reverse complementary strand product due to mispriming to the synthesized cDNA, or the like). In both the TS/TAS-Seq method and the nonTS/TAS-Seq method, the target RNA-capturing step encompasses a solid-phase system in which the target RNA is captured by a target RNA-capture oligo bound to a solid phase such as a bead, and a liquid-phase system in which the target RNA is captured by a free target RNA-capture oligo not bound to a solid phase. Fig. 1 is a scheme of the liquid-phase nonTS/TAS-Seq method. Figs. 2 to 5 are schemes of the TS/TAS-Seq method, wherein Fig. 2 and Fig. 3 illustrate solid-phase systems, and Fig. 4 and Fig. 5 illustrate liquid-phase systems.

Each step is described below for the solid-phase TS/TAS-Seq method, the liquid-phase TS/TAS-Seq method, and the nonTS/TAS-Seq method in this order with reference to drawings as appropriate.

### A. Solid-Phase TS/TAS-Seq Method

### A-1. Target RNA-Capturing Step (Step 1 in Fig. 2 and Fig. 3)

In the target RNA-capturing step of the solid-phase TS/TAS-Seq method, a target RNA is captured by a target RNA-capture oligo bound on a solid-phase carrier. The target RNA is captured on the solid-phase carrier via the target RNA-capture oligo. As described later, the target RNA-capturing step is carried out in a microwell or a microdroplet, or in a solution that does not correspond to these.

The solid-phase carrier may be a bead, or a plate such as a slide glass. Known examples of the single-cell analysis system using a bead solid-phase carrier include the BD Rhapsody System manufactured by Beckton Dickinson (Patent Document 3), the Seq-well System (Non-Patent Document 12) and the Nx1-seq System (Patent Document 5, Non-Patent Document 14), which use a plate having microwells; and the Drop-seq method (Non-Patent Document 13), and the Nadia system manufactured by Dolomite Bio, in which mRNA is captured in a microdroplet. Further, as a system using a plate as a solid-phase carrier, a platform for the spatial transcriptome method is known, wherein a tissue sample is subjected to permeabilization treatment on a plate on which a target-capture oligo is aligned and immobilized, and mRNA derived from each cell of the tissue sample is captured by the capture oligo on the plate. The Stereo-seq system (Chen et al. Cell 2022 185, 1777-1792. doi: 10.1016/j.cell.2022.04.003.) is one example of such a platform. The solid-phase TS/TAS-Seq method is applicable to any of such known techniques.

The material of the bead is not limited, and various materials used for nucleic acid-capturing carriers in nucleic acid analysis kits or for solid-phase particle carriers in immunoassay kits may be employed. Regarding the material of the bead, examples of the bead include beads made of an organic polymer, such as beads made of a resin, for example, polystyrene or polypropylene; beads made of a semiconductor, such as quantum dots (semiconductor nanoparticles) made of a semiconductor material, for example, cadmium selenide (CdSe), zinc sulfide (ZnS), cadmium sulfide (CdS), zinc selenide (ZnSe), or zinc oxide (ZnO); beads made of a metal such as gold; and polymer beads such as beads made of silica. Specific examples of the beads include beads made of a material such as cellulose, a cellulose derivative, an acrylic resin, glass, silica gel, polystyrene, gelatin, polyvinylpyrrolidone, a copolymer of vinyl and acrylamide, divinylbenzene crosslinked polystyrene or the like (see Merrifield Biochemistry 1964, 3,1385-1390), polyacrylamide, latex gel, polystyrene, dextran, rubber, silicon, plastic, nitrocellulose, cellulose, natural sponge, silica gel, glass, metal-plastic, cellulose, cross-linked dextran (including Sephadex (trade name)), or agarose gel (Sepharose (trade name)). Although the bead may be either a non-magnetic body or a magnetic body, a magnetic bead is preferably used since it can be more easily handled.

The material of the plate is also not limited, and, for example, various materials used for microarray substrates may be employed. Representative examples of the materials that may be used include glass, and also silicon and plastics. A plate solid-phase carrier encompasses various solid-phase carriers in the form of a plate having a flat or irregular surface, such as microwell plates on which microwells are aligned in order, plates for use as a substrate for microarray or the like, and flow cells for sequencers. In cases where the solid-phase carrier is a plate, the target RNA-capture oligo is usually immobilized on a plurality of compartments on the plate (typically immobilized such that aligned spots are formed).

In one mode of the target RNA-capturing step in a solid-phase system, an scRNA-seq analysis platform using a solid-phase bead is employed. In this mode, RNA is extracted from each single cell in a microwell or microdroplet, and brought into contact with a bead carrier to which a target RNA-capture oligo is bound. By this, poly(A) RNA or mRNA as a target RNA is captured on the bead within the microwell or microdroplet. In the terms "microwell" and "microdroplet", the term "micro-" typically means that the volume is about 5 to 100 pl. Examples of a common method for an scRNA-seq analysis platform using a solid-phase bead are as described above, and include the BD Rhapsody System manufactured by Beckton Dickinson (Patent Document 3), the Seq-well System (Non-Patent Document 12), and the Nx1-seq System (Patent Document 5, Non-Patent Document 14), which use a plate having microwells; and the Drop-seq method (Non-Patent Document 13), and the Nadia system manufactured by Dolomite Bio, in which mRNA is captured in a microdroplet. These common methods may be preferably used in the present invention.

In another mode of the target RNA-capturing step in a solid-phase system, a platform for the spatial transcriptome method is used. In this mode, a tissue sample is subjected to permeabilization treatment on a plate on which a target RNA-capture oligo is aligned and immobilized, and poly(A) RNA or mRNA leaked out of each cell of the tissue sample is captured with the capture oligo on the plate. One example of such a platform is the Stereo-seq system (Chen et al. Cell 2022 185, 1777-1792. doi: 10.1016/j.cell.2022.04.003.). This is a mode in which the target RNA is captured in a solution whose volume does not fall within the above-described definition of "micro-".

In still another mode of the target RNA-capturing step in a solid phase, a poly(A) RNA sample or an mRNA sample is prepared from a cell sample such as a lesion tissue specimen or a cell suspension, and a solid-phase carrier to which a poly(A) RNA-capture oligo is bound is brought into contact with the poly(A) RNA sample or the mRNA sample, to capture poly(A) RNA on the solid-phase carrier. This is a mode in which the target RNA is captured in a solution whose volume does not fall within the above-described definition of "micro-". In this mode, in the preparation of the poly(A) RNA sample or the mRNA sample, first, total RNA may be extracted from the cell sample, and poly(A) RNA or mRNA may then be extracted therefrom. Or, poly(A) RNA or mRNA may be directly extracted from the cell sample without carrying out total RNA extraction.

The target RNA in the TS/TAS-Seq method contains poly(A) RNA. Poly(A) RNA alone may be targeted, or an RNA other than poly(A) RNA may also be targeted together. In other words, in the TS/TAS-Seq method, the target RNA-capture oligo contains a target poly(A) RNA-capture oligo, and may additionally contain a target RNA-capture oligo that captures an RNA other than poly(A) RNA. Further, in addition to the target RNA-capture oligo, a target capture oligo that captures a nucleic acid molecule other than RNA as a target may be contained. One kind, or two or more kinds of target RNA-capture oligos may be immobilized on the solid phase, and in addition, a target capture oligo that captures a nucleic acid molecule other than RNA may also be immobilized on the solid phase. Examples of the target capture oligo that captures a nucleic acid molecule other than RNA include an oligonucleotide that captures a labeled oligo DNA in an scRNA-seq analysis technique combined with an oligo DNA-labeled antibody, such as TotalSeq manufactured by BioLegend. A technique combining such an oligo DNA-labeled antibody with the TdT method is also known (Patent Document 2). Another example of the target capture oligo that captures a nucleic acid molecule other than RNA is an oligonucleotide that captures a DNA fragment recombined by Tn5 transposase in a single-cell chromatin structure analysis technique combined with Tn5 transposase. In a mode in which a plurality of kinds of capture oligos are used in combination, when the solid phase is a bead, a plurality of kinds of capture oligos may be immobilized on one bead, or a plurality of kinds of capture oligos may be separately immobilized on different beads. When the solid phase is a plate, a plurality of kinds of capture oligos may be immobilized in one compartment, or a plurality of kinds of capture oligos may be separately immobilized in different compartments.

A typical example of the target poly(A) RNA-capture oligo is an oligonucleotide comprising a poly(T) portion as the target capture portion, which captures a poly(A) portion of the target RNA. Examples of a target RNA-capture oligo that captures a portion other than poly(A) include oligonucleotides containing a random sequence of 6 to 15 bases as a target capture portion, and oligonucleotides comprising a sequence complementary to a desired region in a certain RNA sequence (for example, the T-cell receptor constant region, the B-cell receptor constant region, a region in the vicinity of the 5'-end of RNA, or the like) as a target capture portion.

The 5'-end of the target RNA-capture oligo is directly or indirectly bound to a solid-phase carrier, and the oligo has a target capture portion at the 3'-end. The oligo may comprise a first adapter portion on the 5'-side, that is, the solid-phase carrier side, of the target capture portion. The portion represented as Universal 1 in Fig. 2 and Fig. 3 is the first adapter portion. The first adapter portion serves as a region in which one of the primers in the nucleic acid amplification step is set.

The target RNA-capture oligo may comprise, between the first adapter portion and the target capture portion, a barcode portion for identifying each solid-phase carrier or each position on the solid-phase carrier. In cases where the solid-phase carrier is a bead, the barcode portion is a bead identification barcode, and in cases where the solid-phase carrier is a plate, the barcode portion is a compartment identification barcode for identifying which compartment on the plate the target RNA-capture oligo is immobilized on. Thus, the target RNA-capture oligo may comprise, from the 5'-side to the 3'-side, a first adapter portion, a bead or compartment identification barcode portion, and a target capture portion. In a mode in which such a barcode portion is included, the complementary DNA strand amplified in the nucleic acid amplification step comprises a barcode sequence derived from the identification barcode portion. Bead identification barcode portions have the same sequence on the same bead, and have different sequences from one bead to another, by which cDNAs derived from mRNAs captured on the same bead can be distinguished from cDNAs derived from mRNAs captured on other beads. In scRNA-seq analysis, cDNAs derived from the same cell can be distinguished from cDNAs derived from other cells. A solid-phased poly(A) RNA-capture oligo comprising such a bead identification barcode portion can be prepared by a known method (see, for example, WO 2015/166768 A1). Compartment identification barcode portions have the same sequence when they are in the same compartment (spot) on a plate, and have different sequences from one compartment (spot) to another, by which, for example, what kind of mRNA is expressed in which site of the tissue can be understood when the sample is a tissue specimen.

Usually, a large number of target RNA-capture oligo molecules are immobilized on a single bead or within a single compartment. Fig. 2 and Fig. 3 show modes wherein the solid phase is a bead, wherein the target RNA-capture oligo is a target poly(A) RNA-capture oligo comprising a poly(T) portion as a target capture portion, and wherein mRNA is captured as the targe poly(A) RNA (part of the poly(T) portion in the capturing oligo is not shown in the figures). One mRNA molecule captured on one bead carrier is drawn for convenience. However, in practice, a large number of mRNA molecules are captured on one bead carrier. Not only full-length mRNAs having a 5' cap structure, but also "mRNAs with an incomplete length" such as non-full-length mRNAs, immature mRNAs and partially degraded mRNAs that have no cap structure are captured on the solid phase by the target poly(A) RNA-capture oligo as long as they have poly(A).

### A-2. Complementary-Strand Synthesis Step (Step 2 in Fig. 2 and Fig. 3)

After the target RNA-capturing step, the solid-phase carrier is washed, and the complementary-strand synthesis step is carried out in the state where the target RNA is captured on the solid-phase carrier. In the complementary-strand synthesis step in TS/TAS-Seq, reverse transcription reaction is carried out using a reverse transcriptase having terminal transferase activity in the presence of a template-switching oligo, to carry out the reverse transcription reaction and template-switching reaction simultaneously (in one step). The timing of addition of the template-switching oligo into the reaction system is not limited. The template-switching oligo may be added before the beginning, at the same time as the beginning, or after the beginning of the reverse transcription reaction, or may be added after a certain length of time during the reverse transcription reaction. The reverse transcription reaction and the template-switching reaction, per se, may be carried out according to conventional methods.

Reverse transcriptases having terminal transferase activity are widely known, and a variety of such reverse transcriptases are commercially available. Representative examples of such reverse transcriptases include wild-type MMLV RT; SuperScript II, SuperScript IV, and Maxima H minus reverse transcriptase, manufactured by Thermo Fisher Scientific; SmartScribe reverse transcriptase, manufactured by Takara Bio Inc.; and template switching RT, manufactured by New England Biolabs.

By the reverse transcription reaction, from the target RNA captured on the solid-phase carrier, a DNA strand whose sequence is complementary thereto (complementary DNA strand, first strand) is synthesized. Subsequently, by the terminal transferase activity of the reverse transcriptase, an additional sequence consisting of several arbitrary bases is added to the 3'-end of at least part of the complementary DNA molecules synthesized. In the template-independent base addition reaction to the 3'-end by the terminal transferase activity, it is known that the number of bases added is generally within the range of one to five bases, and that three bases are often added. It is also known that, in cases where the RNA that serves as the template is a full-length mRNA having a cap structure at the 5'-end, CCC tends to be added at the 3'-end of the cDNA. The template-switching oligo is an oligonucleotide comprising, at its 3'-end, a sequence capable of hybridizing with the additional sequence, and also comprising a switching sequence on the 5'-side of the sequence capable of hybridizing with the additional sequence. Therefore, in this step, the template-switching oligo hybridizes with the complementary DNA strand to which the additional sequence has been added, and the complementary DNA strand extends to the 5'-end of the template-switching oligo, to further add the complementary strand of the switching sequence to the 3'-end of the complementary DNA.

The template-switching oligo preferably comprises an oligonucleotide comprising, at its 3'-end, GGG, GUG, or NGG (wherein G and U are bases of ribonucleotides, and N is a base of any ribonucleotide selected from A, U, G, and C; and wherein one or more nucleotide analogues are optionally included) as the sequence capable of hybridizing with the additional sequence. Fig. 2 and Fig. 3 show cases where the hybridizable 3'-end sequence is the ribonucleotide bases of GGG. A mixture of two or more template-switching oligos having different 3'-end sequences may be used.

The switching sequence may comprise a second adapter portion. The portion represented as Universal 2 in the figures is the second adapter portion. The second adapter portion serves as a region in which one of the primers in the nucleic acid amplification step, which is carried out later, is set.

The switching sequence may comprise a molecular barcode portion consisting of a random sequence on the 3'-side of the second adapter portion (between the second adapter portion and the 3'-end sequence described above in the template-switching oligo). Among the DNA molecules amplified by the nucleic acid amplification step, which is carried out later, DNAs derived from the same double-stranded cDNA have the same molecular barcode, while DNAs derived from different double-stranded cDNAs have molecular barcodes that are different from each other. By counting only DNAs having different molecular barcodes, assumption of the number of cDNA molecules at the start, and correction of variation of the PCR amplification efficiency among different cDNAs are possible. Although the chain length of the molecular barcode is not limited, it is typically about 12 bases to 30 bases. To make the molecular barcode tolerant of sequencing errors, the molecular barcode may be configured such that a certain fixed sequence is inserted in the random base sequence. As described later, the long primer used as the second-strand-synthesizing primer in the second-strand synthesis step may also comprise a similar molecular barcode portion.

The template-switching oligo may comprise, at the 5'-end and/or the 3'-end (either one or both) of the switching sequence, a chemical modification or a base for preventing the polymerization of the template-switching oligo, such as amino modification, biotin modification, or C3 spacer modification.

As shown in the first row in Step 2 in Fig. 2 and Fig. 3, when the target RNA-capture oligo has captured a full-length mRNA and cDNA has been synthesized for the full-length mRNA, CCC is added to the 3'-end of the cDNA with a high probability by the terminal transferase activity of the reverse transcriptase. In cases where a template-switching oligo whose 3'-end sequence is GGG has hybridized with the CCC portion, the DNA-dependent DNA polymerase activity of the reverse transcriptase causes synthesis of the complementary strand of the switching sequence directly after the CCC at the 3'-end of the cDNA (template-switched cDNA). In the illustrated examples, the complementary strand of the second adapter, Universal 2, is synthesized. However, when a molecular barcode is contained between Universal 2 and rGrGrG in the template-switching oligo, the molecular barcode complementary strand + the Universal 2 complementary strand are synthesized directly after the CCC at the 3'-end of the cDNA. As a result of the synthesis of the complementary strand of the switching sequence, the mRNA-cDNA hybrid has a blunt end. Therefore, several arbitrary bases (represented as NNN in the figures) may be further added (or may not be added) to the 3'-end of the cDNA strand by the terminal transferase activity of the reverse transcriptase.

Even when cDNA has been synthesized for the entire full-length mRNA and CCC has been added to its 3' end, cDNA molecules with which a template-switching oligo has not been hybridized may occur. In addition, in some cases, an additional sequence with which a template-switching oligo cannot hybridize may be added to the 3' end of the cDNA that have been synthesized for the entire full-length mRNA. Such cDNA molecules remain on the solid phase as cDNA that has not undergone the template-switching reaction (second row in Step 2, non-switched cDNA).

On a target RNA-capture oligo that has captured an mRNA with an incomplete length, several arbitrary bases (NNN) other than CCC tend to be added (or may not be added) to the end of the synthesized cDNA. Therefore, such a cDNA also remains on the solid phase as a cDNA that has not undergone the template-switching reaction (third row in Step 2, non-switched cDNA).

When full-length mRNA is captured but the cDNA synthesis has stopped midway, the cDNA end of the mRNA-cDNA hybrid becomes a recessed end. In such cases, base addition to the cDNA end does not occur, so that the cDNA remains on the solid phase without undergoing the template-switching reaction (fourth row in Step 2, non-switched cDNA).

In the complementary-strand synthesis step, it is also possible to use a reverse transcriptase having strand displacement activity in addition to terminal transferase activity. In this case, as shown in Step 2 in Fig. 3, in the hybrid double strand of the template-switched cDNA and mRNA, the second-strand synthesis proceeds while the mRNA is stripped from the cDNA by the strand displacement activity of the reverse transcriptase.

Reverse transcriptases having terminal transferase activity and strand displacement activity are also known, and a variety of such reverse transcriptases are commercially available. Representative examples of such reverse transcriptases include wild-type MMLV RT; SuperScript II, SuperScript IV, and Maxima H minus reverse transcriptase, manufactured by Thermo Fisher Scientific; SmartScribe reverse transcriptase, manufactured by Takara Bio; and template switching RT, manufactured by New England Biolabs.

### A-3. Unreacted-Oligo Removal Step (Step 3 in Fig. 2 and Fig. 3)

After the complementary-strand synthesis step, the solid-phase carrier is washed, and the unreacted-oligo removal step is carried out. In this step, target RNA-capture oligos that remain on the solid phase and have not captured any target RNA (unreacted capture oligos) are removed. In the solid-phase system, template-switching oligos that remain in the reaction liquid and have not hybridized with the complementary DNA strands (unreacted template-switching oligos) are removed by the washing of the solid phase. Therefore, unreacted capture oligos on the solid phase are degraded and removed by exonuclease treatment. Common examples of exonucleases that specifically degrade single-stranded DNA include exonuclease I and exonuclease T. In the present invention, at least one of such common exonucleases may be used. After the exonuclease treatment, the solid-phase carrier is washed, and then the following step is carried out.

### A-4. RNA Degradation Step (Step 4 in Fig. 2 and Fig. 3)

In the RNA degradation step, the mRNA in the mRNA-cDNA hybrid bound on the solid-phase carrier is degraded with a ribonuclease. In cases where the 3'-end sequence of the template-switching oligo is ribonucleotide bases, this 3'-end portion is also degraded. In this step, a common ribonuclease such as RNase H may be used, or alkali treatment with an NaOH solution, a KOH solution, or the like may be used. After the degradation of the RNA, the solid-phase carrier may be washed and collected, followed by proceeding to the homopolymer addition step, or may be subjected as it is to the homopolymer addition step without washing. As described later, the RNA degradation step and the homopolymer addition step may be carried out simultaneously.

### A-5. Homopolymer Addition Step (Step 4 in Fig. 2 and Fig. 3)

In the homopolymer addition step, reaction by terminal deoxynucleotidyl transferase (TdT) is carried out in the presence of dATP, dTTP, dCTP, or dGTP, and a chain-terminating nucleotide triphosphate (chain-terminating NTP), to add a nucleotide homopolymer to the 3'-end of the complementary DNA strand synthesized on the solid phase (Step 4 in Fig. 2 and Fig. 3; the figures show an example with poly(C)). The nucleotide homopolymer is added to both template-switched cDNAs and non-switched cDNAs. The chain-terminating NTP (chain-terminating ATP, chain-terminating TTP, chain-terminating CTP, or chain-terminating GTP) is, as is well known in the art, a nucleotide modified or altered such that the OH group at the 3'-position of the nucleotide cannot form a phosphoester bond with the 5'-phosphate portion of another nucleotide molecule. The chain-terminating NTP can also be said to be a nucleotide triphosphate having no OH group at the 3'-position (comprising no OH group in the atomic group bound to the 3'-position). Specific examples of common chain-terminating NTPs that can also be used in the present invention include dideoxynucleotide triphosphates (ddNTPs) (ddATP, ddCTP, ddGTP, ddTTP, and ddUTP), ddNTP derivatives (typically ddNTPs whose 3'-position is modified with an atomic group having no OH group, such as ddNTPs whose 3'-position is modified with an azido group or an amino group, for example, 3'-azido-ddATP, 3'-azido-ddCTP, 3'-azido-ddGTP, 3'-azido-ddTTP, 3'-azido-ddUTP, 3'-amino-ddATP, 3'-amino-ddCTP, 3'-amino-ddGTP, and 3'-amino-ddTTP), 3'-deoxynucleotide triphosphates (3'-dNTPs) (3'-dATP, 3'-dCTP, 3'-dGTP, 3'-dTTP, and 3'-dUTP), and 3'-deoxy-5-methyluridine-5'-triphosphate. The chain-terminating NTP may be, for example, ddNTP, or ddNTP whose 3'-position is modified with an azido group, or may especially be ddNTP, although the chain-terminating NTP is not limited thereto. It is preferred that the base chain of the chain-terminating NTP should match the base chain of the homopolymer-forming substrate (dNTP). It is preferable to perform poly(C) addition or poly(G) addition using a combination of chain-terminating CTP, e.g. ddCTP, + dCTP or a combination of chain-terminating GTP, e.g. ddGTP, + dGTP, because it is possible to advantageously shorten the minimum homopolymer length required for the second-strand synthesis reaction, the necessary length of the 3'-end homopolymer portion of the primer used in the second-strand synthesis, and the chain lengths of by-products derived from free primers. In the TS/TAS-Seq method, which uses the TS reaction in combination, the cytosine bases added to the 3'-end of the cDNA strand for the full-length mRNA in the TS reaction can also be counted as the homopolymer portion, and therefore it is especially preferred to perform poly(C) addition by the combination of chain-terminating CTP + dCTP.

For example, in cases where ddNTP is used, the amount of the chain-terminating NTP added, in terms of the ratio to the dNTP (dATP, dTTP, dCTP, or dGTP), may be ddNTP:dNTP = about 1:10 to 1:100. The chain-terminating NTP may be used at a ratio of, for example, 1:10 to 1:80, 1:10 to 1:60, 1:10 to 1:40, 1:15 to 1:80, 1:15 to 1:60, or 1:15 to 1:40. The amounts of other chain-terminating NTPs may also be set in accordance with this ratio. In cases of a conventional TdT method in which the TdT reaction is carried out in the absence of the chain-terminating NTP, the reaction time needs to be strictly controlled for controlling the chain length of the homopolymer in accordance with the lot-to-lot variation of the activity of the enzyme, and the differences in the amounts of the primers and substrate such as cDNA. In contrast, in the TAS-Seq method developed by the present inventors, since the homopolymer addition reaction by TdT is carried out in the presence of the chain-terminating NTP, the chain-terminating NTP is stochastically incorporated to stop the homopolymer extension reaction, so that the acceptable degree of variation in the TdT reaction time and in the enzyme activity can be largely increased.

The homopolymer addition reaction by TdT is generally carried out in the presence of a divalent cation. Examples of the divalent cations that may be used include divalent metal cations such as Zn²⁺, Cu²⁺, Ni²⁺, Co²⁺, Mn²⁺, and Mg²⁺. For example, the cation may be, but is not limited to, Co²⁺, Mn²⁺, or Mg²⁺.

Since the addition of the chain-terminating NTP largely increases the acceptable degree of variation of the TdT reaction time and the like, RNase H can be added to the TdT reaction liquid to carry out the RNA degradation reaction and the homopolymer addition reaction simultaneously. Thus, in one mode of the present invention, the RNA degradation step and the homopolymer addition step are carried out simultaneously.

### A-6. Second-Strand Synthesis Step (Step 5 in Fig. 2 and Fig. 3)

After the homopolymer addition reaction, the solid-phase carrier is washed, and then the second-strand synthesis step is carried out. The second-strand-synthesizing primer used in the second-strand synthesis step of the TS/TAS-Seq method comprises: a primer comprising a complementary homopolymer portion whose sequence is complementary to the nucleotide homopolymer added to the end of the first strand of the complementary DNA, and also comprising a second adapter portion on the 5'-side of the complementary homopolymer portion (hereinafter referred to as long primer); and a primer comprising no complementary homopolymer portion, and comprising a second adapter portion at its 3'-side region (hereinafter referred to as short primer). The template-switched cDNA derived from the full-length mRNA comprises the complementary strand of the second adapter (Universal 2) derived from the template-switching oligo. Therefore, both the long primer and the short primer can hybridize with the template-switched cDNA. In general, a primer located inside is extended more readily. Therefore, the figures illustrate extension from the short primer. With a non-switched complementary DNA strand, the long primer hybridizes, to allow extension and synthesis of the second strand. By the second strand synthesis, a DNA double strand composed of the complementary DNA (first strand) and the second strand is formed on the solid phase. In cases where the template-switching oligo comprises a molecular barcode, the molecular barcode is incorporated into the second strand that is synthesized for the template-switched first-strand cDNA in the present step.

The complementary homopolymer portion is present at the 3'-end region of the long primer. In cases where the homopolymer tail added to the first strand complementary DNA is poly(C) or poly(G), the chain length of the complementary homopolymer portion is usually about 6 to 15 bases, for example, about 7 to 13 bases. In cases where the homopolymer tail is poly(A) or poly(T), the chain length is usually about 15 to 30 bases, for example, about 18 to 25 bases. To the 3'-end of the complementary homopolymer portion, an anchor sequence of about 1 to 2 bases may be added. By the addition of the anchor sequence, the probability of annealing of the long primer to the start point of the homopolymer tail that has been added to the cDNA by TdT can be increased. As the anchor sequence, H or HN (H = A, T, or C; N = any base (A, T, C, or G)) may be used when the complementary homopolymer portion is poly(G); D or DN (D = A, T, or G; N = any base (A, T, C, or G)) may be used when the complementary homopolymer portion is poly(C); B or BN (B = T, G, or C; N = any base (A, T, C, or G)) may be used when the complementary homopolymer portion is poly(A); or V or VN (V = A, G, or C; N = any base (A, T, C, or G)) may be used when the complementary homopolymer portion is poly(T).

The second adapter contained in the long primer and the short primer is the same as the second adapter contained in the template-switching oligo, and serves as a region in which one of the primers is set in the nucleic acid amplification step, which is carried out later.

Similarly to the template-switching oligo, the long primer may comprise a molecular barcode portion consisting of a random sequence. The long primer may comprise a molecular barcode between the second adapter portion and the complementary homopolymer portion. In an amplification product derived from a template-switched cDNA, the molecular barcode contained in the switching sequence of the template-switching oligo functions. In an amplification product derived from a non-switched cDNA, the molecular barcode given to the long primer functions.

The polymerase used in the second-strand synthesis step may be a common polymerase such as Taq polymerase used in ordinary PCR, or may be a polymerase known as a high-fidelity PCR enzyme, or a polymerase having strand displacement activity such as Bst DNA polymerase.

In the second-strand synthesis step, heat denaturation may be carried out when necessary, and then annealing of the second-strand-synthesizing primer and the extension reaction may be carried out in only one cycle, or in cycles by the thermal cycling reaction. Further, after carrying out the reaction in one cycle or a plurality of cycles, a polymerase having strand displacement activity such as Bst DNA polymerase may be used to carry out follow-up reaction.

### A-7. Nucleic Acid Amplification Step (Step 6 in Fig. 2 and Fig. 3)

After the second strand synthesis, the solid-phase carrier is washed or not washed, and then the nucleic acid amplification step is carried out. In the nucleic acid amplification step, nucleic acid amplification reaction is carried out using, as a template, the DNA double strand synthesized on the solid-phase carrier (Step 6 in Fig. 2 and Fig. 3). Since this nucleic acid amplification reaction requires high-fidelity, it is preferred to use a polymerase generally known as a high-fidelity PCR enzyme. Various high-fidelity PCR enzymes are commercially available. In this step, a nucleic acid amplification reaction liquid can be added without washing the solid-phase carrier, to directly carry out the nucleic acid amplification reaction.

In a mode employing a first adapter and a second adapter, nucleic acid amplification reaction may be carried out using a set of primers set in those adapters. In cases of scRNA-seq analysis, mRNAs expressed in each cell are comprehensively reverse transcribed and amplified. Therefore, it is common to carry out total cDNA amplification with a primer set targeting the first and second adapters. Although the primer set in this case is typically a set of a primer consisting of the first adapter sequence and a primer consisting of the second adapter sequence, such primers that comprise an arbitrary additional sequence or modification with biotin or the like in its 5'-side region can also be used.

Or, a primer targeting a desired region in the complementary DNA strand may be used instead of the primer targeting the first adapter, to carry out the nucleic acid amplification reaction. In cases where the amplification may be carried out for only a specific region in the complementary DNA molecule synthesized on the solid-phase carrier, for example, in the case of the preparation of a TCR variable-region library in T-cell receptor (TCR) repertoire analysis, a primer targeting the second adapter and a primer targeting a desired region in the complementary DNA strand (for example, a primer targeting the TCR constant region) may be used to carry out the nucleic acid amplification reaction.

The nucleic acid amplification reaction itself may be carried out by PCR according to an ordinary method. If desired, the PCR reaction may be carried out in one step, or in two or more steps. For example, 1st PCR may be carried out for several cycles using a primer set targeting the first and second adapters, and then size selection and purification of the amplification product may be carried out, followed by carrying out 2nd PCR for several ten cycles using a primer set targeting the same site, or using a set of a primer targeting the second adapter and a primer targeting a partial region positioned inside relative to the first adaptor. Thereafter, size selection and purification of the amplification product may be carried out again to obtain an amplification product of total cDNA. In the 2nd PCR, each primer may comprise a spacer such as biotin or amine bound to its 5'-end. By this, the DNA fragments to be subjected to the sequence analysis can be easily collected.

### B. Liquid-Phase TS/TAS-Seq Method

### B-1. Target RNA-Capturing Step (Step 1 in Fig. 4 and Fig. 5)

In the target RNA-capturing step of the liquid-phase TS/TAS-Seq method, a target RNA is captured by a free target RNA-capture oligo not bound on a solid-phase carrier. Also in the liquid-phase system, the target RNA-capturing step may be carried out in a microwell or a microdroplet, or in a solution that does not correspond to these.

Known examples of the liquid-phase analysis technique include the 10X Chromium system, manufactured by 10X Genomics, in which a single cell and a hydrogel bead containing mRNA-capture oligos encapsulated therein are encapsulated in a microdroplet, and melting of the hydrogel and lysis of the cell are allowed to occur in the microdroplet to capture mRNA with the released mRNA-capture oligos (Patent Document 4); Smart-Seq2, in which amplification is performed after dispensing individual cells or a minute amount of mRNA on a 96-well plate (Non-Patent Document 15); and sci-RNA-seq3, in which fixed cells are suspended in a solution containing a cell membrane permeabilization reagent and an mRNA-capture oligos, and mRNA is captured on the mRNA-capture oligos in the fixed cells. The liquid-phase TS/TAS-Seq method is applicable to any of such known transcriptome analysis techniques.

In one mode of the target RNA-capturing step in the liquid-phase system, target RNA-capture oligos are encapsulated in a bead made of a material that can be melted under mild conditions, for example, at room temperature (such as a hydrogel), and the bead and a cell, on a one-to-one basis, are encapsulated in a microdroplet or added to a microwell, followed by allowing melting of the bead and lysis of the cell in the microdroplet or microwell, to capture target RNA such as mRNA on the target RNA-capture oligos.

In another mode, target RNA-capture oligos are added to a solution containing a cell lysate or RNA extracted from a cell, and a target RNA such as mRNA is captured on the capture oligos in the solution. This mode encompasses a method in which each cell or mRNA extracted therefrom is dispensed into a microwell, and a target RNA such as mRNA is captured on target RNA-capture oligos in the microwell, a method in which each cell is trapped in a microdroplet, and a target RNA is captured on target RNA-capture oligos in the microdroplet, and a method in which a target RNA is captured on target RNA-capture oligos in a solution whose volume does not correspond to a microvolume.

In another mode, fixed cells are suspended in a solution containing a cell membrane permeabilization reagent and target RNA-capture oligos, and a target RNA such as mRNA is captured on the target RNA-capture oligos in the fixed cells.

The target RNA comprises poly(A) RNA as described for the solid-phase system, and this also applies to the liquid-phase TS/TAS-Seq method. Poly(A) RNA alone may be targeted, or RNA other than poly(A) RNA may also be targeted together. In other words, in the TS/TAS-Seq method, the target RNA-capture oligo contains a target poly(A) RNA-capture oligo, and may additionally contain a target RNA-capture oligo that captures RNA other than poly(A) RNA. Further, in addition to the target RNA-capture oligo, a target capture oligo that captures a nucleic acid molecule other than RNA as a target may be contained. In cases where the target RNA is captured in a microwell or a microdroplet, plural kinds of capture oligos may be contained in one microwell or microdroplet, or plural kinds of capture oligos may be separately contained in different microwells or microdroplets.

Typical examples of the target poly(A) RNA-capture oligo, and examples of the target RNA-capture oligo that captures a portion other than poly(A) are the same as those for the solid-phase TS/TAS-Seq method. As shown in Step 1 in Fig. 4 and Fig. 5, not only full-length mRNAs having a 5' cap structure, but also "mRNAs with an incomplete length" such as non-full-length mRNAs, immature mRNAs, and partially degraded mRNAs that have no cap structure are captured by a target poly(A) RNA-capture oligo having a T portion as a target capture portion, as long as they have poly(A).

The target RNA-capture oligo has a target capture portion at its 3'-end. On the 5'-side of the target capture portion, a first adapter portion that can be used as a primer setting region in the nucleic acid amplification step (represented as Universal 1 in Fig. 4 and Fig. 5) may be contained. Between the first adapter portion and the target capture portion, a cell identification barcode portion for identifying each cell from which the target RNA is derived may be contained. The cell identification barcode is a barcode corresponding to the bead identification barcode portion and the compartment identification barcode portion in the solid-phase system. The target RNA-capture oligos to be encapsulated together with one cell in the same microwell or microdroplet have a cell identification barcode consisting of the same sequence, and the cell identification barcode sequence is different among microwells or microdroplets. Therefore, cDNAs derived from mRNAs captured by the capture oligos in the same microwell or microdroplet can be distinguished from cDNAs derived from mRNAs captured in other microwells or microdroplets, and hence cDNAs derived from the same cell can be distinguished from cDNAs derived from other cells.

### B-2. Complementary-Strand Synthesis Step (Step 2 in Fig. 4 and Fig. 5)

In a liquid-phase system, the target RNA-capturing step is directly followed by the complementary-strand synthesis step, without carrying out purification and the like. To the reaction liquid after the target RNA-capturing step, a reverse transcriptase having terminal transferase activity and template-switching oligos are added to carry out the reverse transcription reaction and the TS reaction in one step.

Details of this step, and the structure of the template-switching oligo are the same as those for the solid-phase TS/TAS-Seq. The first row in Step 2 in Fig. 4 and Fig. 5 is a template-switched cDNA. cDNAs which have been synthesized along the entire full-length mRNA, but with which the template-switching oligo has not hybridized (the second row in Step 2 in Fig. 4 and Fig. 5), cDNAs which have been synthesized along the entire mRNA with an incomplete length, and with which the template-switching oligo has not hybridized (the third row in Step 2), and cDNAs whose synthesis has stopped midway (the fourth row in Step 2) remain in the reaction liquid as cDNAs that have not undergone the template-switching reaction.

When a reverse transcriptase having strand displacement activity in addition to terminal transferase activity is used, in the hybrid double strand of the template-switched cDNA and mRNA, the second-strand synthesis proceeds while the mRNA is stripped from the cDNA by the strand displacement activity of the reverse transcriptase as shown in Step 2 in Fig. 5. A difference from the solid-phase system is that the 3'-end of the synthesized second strand and the 5'-end of the cDNA strand form a blunt end, and hence that bases may be added (or may not be added) also to the 3'-end of the second strand by the terminal transferase activity of the reverse transcriptase.

### B-3. Unreacted-Oligo Removal Step (Step 3 in Fig. 4 and Fig. 5)

In the case of a liquid-phase system, the reaction liquid after the completion of the complementary-strand synthesis may be purified by performing nucleic acid size fractionation, to separate and remove unreacted target RNA-capture oligos having a very short chain length from the complementary DNA strand (mRNA-cDNA hybrid). Unreacted template-switching oligos can also be removed in one step together with the unreacted capture oligos. Purification methods by nucleic acid size fractionation are well known, and the purification can be carried out according to a conventional method such as purification using a silica membrane column or purification using a magnetic bead to which DNA binds. In this step, the nucleic acid size fractionation may be combined with removal by exonuclease in order to further increase the efficiency of the removal of the unreacted oligos. A common exonuclease that specifically degrades single-stranded DNA may be used, and examples of such an exonuclease include exonuclease I and exonuclease T. After the completion of the complementary strand synthesis, the exonuclease may be added to the reaction liquid to allow the reaction to proceed, and then purification by nucleic acid size fractionation may be carried out to achieve removal of the exonuclease at the same time.

### B-4. RNA Degradation Step (Step 4 in Fig. 4 and Fig. 5)

Ribonuclease is added to the reaction liquid after the purification by size fractionation, to degrade the mRNA of the mRNA-cDNA hybrid and the 3'-end sequence of the template-switching oligo (in cases where the sequence is ribonucleotide bases) in the reaction liquid.

After the RNA degradation by the addition of the ribonuclease, reagents such as dNTP, chain-terminating NTP, and TdT may be added to the reaction liquid to carry out the subsequent homopolymer addition step. However, it is preferred to add the ribonuclease and these reagents to the reaction liquid simultaneously or sequentially to carry out the RNA degradation and the homopolymer addition at the same time. In cases where an alkaline solution of NaOH, KOH, or the like is used in the RNA degradation step, the degradation reaction with the alkaline solution is followed by neutralization of the reaction liquid before carrying out the TdT reaction.

### B-5. Homopolymer Addition Step (Step 4 in Fig. 4 and Fig. 5)

Reaction by TdT is carried out in the presence of dATP, dTTP, dCTP, or dGTP, and a chain-terminating NTP, to add a nucleotide homopolymer to the 3'-end of the complementary DNA strand (Step 4 in Fig. 4 and Fig. 5; the figures show an example with poly(C)). Details of the present step, such as preferred examples of the chain-terminating NTP, the addition ratio between the dNTP and the chain-terminating NTP, etc. are the same as those for the solid-phase TS/TAS-Seq method except for the following point.

Fig. 5 shows a mode where the reverse transcriptase used in the complementary-strand synthesis step also has strand displacement activity. In this mode, the second strand (the strand indicated by the asterisk in Step 4) for the template-switched cDNA is synthesized in the complementary-strand synthesis step. Therefore, a DNA double strand composed of the cDNA first strand and second strand is present in the reaction liquid at the beginning of the homopolymer addition step, and each end of this DNA double strand is a blunt end or a 3'-protruding end (see the first row in Step 2 in Fig. 5). Therefore, in the DNA double strand, the homopolymer may be added not only to the 3'-end of the template-switched cDNA first strand, but also to the 3'-end of the second strand.

### B-6. Second-Strand Synthesis Step (Step 5 in Fig. 4 and Fig. 5)

After the RNA degradation and the homopolymer addition, the reaction liquid is purified to remove reagents such as the ribonuclease and the TdT, and then the second-strand synthesis step is carried out. The second-strand synthesis step of liquid-phase TS/TAS-Seq is carried out using a second-strand-synthesizing primer comprising a long primer and a short primer, similarly to the solid phase system. The configuration of each primer, preferred conditions, and details of this step are the same as those for solid-phase TS/TAS-Seq except for the following point.

A difference from the solid-phase system is that, in the mode using a reverse transcriptase further having strand displacement activity shown in Fig. 5, the second strand synthesized for the template-switched cDNA in the complementary-strand synthesis step (the strand with the asterisk) may also have a homopolymer at its 3'-end, so that a reaction may occur such that the long primer hybridizes with the homopolymer of this strand to allow extension as illustrated in the dotted-line box in Step 5. The DNA strand generated by this extension reaction does not contain the adapter sequence required for the PCR amplification at its 5'-end region. Therefore, this DNA strand is not amplified when primers targeting the first and second adapters are used in the subsequent nucleic acid amplification step.

### B-7. Nucleic Acid Amplification Step (Step 6 in Fig. 4 and Fig. 5)

After the second strand synthesis, the reaction liquid is purified, when necessary, to remove reagents such as excessive primers and polymerase. Thereafter, the nucleic acid amplification step is carried out. The present step in a liquid system may be carried out in the same manner as solid-phase TS/TAS-Seq.

### C. nonTS/TAS-Seq Method (Fig. 1)

Although Fig. 1 shows an example for a liquid-phase system, the method is also applicable to a solid-phase system. It can be carried out in the same manner as in the TS/TAS-Seq method except that, in the complementary-strand synthesis step, the reverse transcription reaction is carried out in the absence of the template-switching oligo, and that a reverse transcriptase having terminal transferase activity is used while it does not need to have strand displacement activity. Each step of the nonTS/TAS-Seq method is described below focusing on the difference from the TS/TAS-Seq method.

### C-1. Target RNA-Capturing Step (Step 1 in Fig. 1)

Also in the nonTS/TAS-Seq method, the main target RNA is mRNA, which is poly(A) RNA. In general, the target RNA-capture oligo preferably comprises a target poly(A) RNA-capture oligo. However, since the method does not use TS reaction that utilizes CCC addition to the cDNA of full-length mRNA, the effect of the present invention can be obtained even in cases where nonTS/TAS-Seq is carried out only for non-poly(A) RNA. Thus, for the nonTS/TAS-Seq method, it is not essential that the target RNA-capture oligos comprise target poly(A) RNA-capture oligos.

### C-2. Complementary-Strand Synthesis Step (Step 2 in Fig. 1)

In the complementary-strand synthesis step, reverse transcription reaction is carried out using a reverse transcriptase having terminal transferase activity in the absence of the template-switching oligo. This results in synthesis of the complementary DNA strand of the mRNA captured by the solid-phased or free target RNA-capture oligo, and addition of several arbitrary bases to the 3'-end of at least part of the complementary DNA strands.

### C-3. Unreacted-Oligo Removal Step (Step 3 in Fig. 1)

In the case of a solid-phase system, this step can be carried out in the same manner as A-3. In the case of a liquid-phase system, this step can be carried out in the same manner as B-3.

### C-4. RNA Degradation Step (Step 4 in Fig. 1)

In the case of a solid-phase system, this step can be carried out in the same manner as A-4. In the case of a liquid-phase system, this step can be carried out in the same manner as B-4.

### C-5. Homopolymer Addition Step (Step 4 in Fig. 1)

In the case of a solid-phase system, this step can be carried out in the same manner as A-5. In the case of a liquid-phase system, this step can be carried out in the same manner as B-5. Also in the nonTS/TAS-Seq method, the homopolymer addition step may be carried out after the RNA degradation, or the homopolymer addition and the RNA degradation may be carried out at the same time.

### C-6. Second-Strand Synthesis Step (Step 5 in Fig. 1)

A second-strand-synthesizing primer comprising a long primer is used. No short primer is required.

### C-7. Nucleic Acid Amplification Step (Step 6 in Fig. 1)

This step can be carried out in the same manner as the nucleic acid amplification reaction in the liquid-phase and solid-phase TS/TAS-Seq methods.

The complementary DNA strand amplified by the method of the present invention including nonTS/TAS-Seq and TS/TAS-Seq may be processed by fragmentation, end repair, A-tailing, addition of a sequencing adapter, and the like for subjecting it to sequencing with a next-generation sequencer. By these processes, a library of cDNA processed into a sequencing construct can be obtained. As the means of the fragmentation, ultrasonic or an enzyme may be used. For the preparation of the sequencing construct, commonly used reagents may be used. Representative examples of the enzyme method include those using a NEBNext UltraII FS kit manufactured by New England Biolabs, or a KAPA HyperPlus kit manufactured by KAPA Biosystems. Examples of ultrasonic fragmentation apparatuses that may be used include Covaris and Bioruptor. Other similar products may also be used. As the sequencing adapter, an appropriate adapter compatible with the next-generation sequencer employed may be used.

Sequencing of the library may be carried out using a sequencer that is generally called a next-generation sequencer. Specific examples of next-generation sequencers that may be preferably used include the Novaseq 6000 system, the Novaseq X system, the Hiseq system, the Nextseq system, the Nextseq 2000 system, the Nextseq 3000 system, and the MiSeq system, manufactured by Illumina, Inc.; the DNBseq T7 system, the DNBseq G400 system, and the DNBseq G40 system, manufactured by MGI; and the Ion S5/Ion S5 XL system, manufactured by Thermo Fisher Scientific.

### EXAMPLES

The present invention is described below more concretely by way of Examples. However, the present invention is not limited to the following Examples.

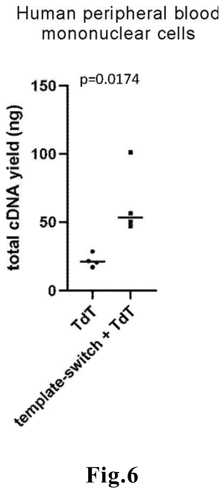

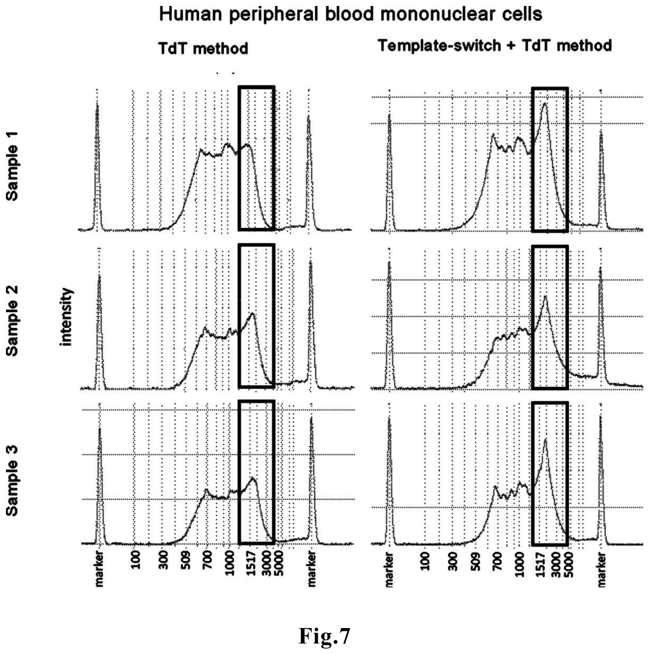

### 1. Comparison between TdT Method (TAS-Seq Method) and cDNA Amplification Method of Present Invention in scRNA-seq Analysis System Using Solid-Phase Carrier (Fig. 6, Fig. 7)

A single-cell transcriptome library trapped on solid-phase beads, prepared by the BD Rhapsody system (Beckton Dickinson), was amplified by the TdT method (TAS-Seq method, Patent Document 2 and Non-Patent Document 11) and a cDNA amplification method according to the present invention (Template-switch + TdT method, or Template-switch + TAS-Seq method), respectively. The sequences of the primers used are shown in Table 1 below.

Human peripheral blood mononuclear cells (PBMCs) that had been cryopreserved after the preparation were obtained from LONZA K. K. The frozen human PBMCs were quickly thawed in a water bath at 37°C, and 9 ml of prewarmed RPMI 1640/5% FBS was added to the suspension, followed by mixing the resulting mixture by inversion, performing centrifugation, removing the supernatant, and then washing the cells again with RPMI 1640/5% FBS. The number of cells was counted using a flow cytometer. The cells and beads were loaded on a microwell cartridge at appropriate densities according to the specification of Rhapsody. The cells were then lysed, and mRNA derived from each single cell was trapped on a single bead (comprising the Rhapsody universal adapter as a poly(A) RNA-capture oligo) (target RNA-capturing step). The Rhapsody universal adapter solid-phased on the beads is DNA having the following structure, wherein 96 kinds of known unique sequences consisting of 9 bases are employed for each of CLS1 (cell label section 1) to CLS3 so that a total of 288 known unique sequences constitute a total of about 900,000 types of bead identification barcode portions. In SEQ ID NO:1 of SEQUENCE LISTING, CLS1 to CLS3 are represented as NNNNNNNNN.

### <Structure of Rhapsody Universal Adapter>

ACACGACGCTCTTCCGATCT-[none or A or GT or TCA]-(CLS1)-GTGA-

(CLS2)-GACA-(CLS3)-NNNNNNNNTTTTTTTTTTTTTTTTTTTTTTTTTTTTTT (SEQ

ID NO:1)

Four tubes containing beads on which mRNA was trapped were each divided into two tubes (a total of eight tubes). Four of the resulting tubes were subjected to the TAS-Seq method, and the remaining four tubes were subjected to the cDNA amplification method of the present invention, to perform the process from reverse transcription to cDNA amplification.

In the TAS-Seq method, the poly(A) RNA-capture beads were subjected to reverse transcription as recommended by the manufacturer (complementary-strand synthesis step; a reverse transcriptase having terminal transferase activity and strand displacement activity was used), and then exonuclease I treatment was carried out to remove unreacted poly(A) RNA-capture oligos on the beads (unreacted-oligo removal step). mRNA degradation by RNase H and homopolymer addition reaction by TdT were carried out simultaneously for 20 minutes using dCTP, and ddCTP as a terminator. After washing the beads, second-strand synthesis reaction was carried out using a primer having a guanine homopolymer at the 3'-end of the universal 2 adapter sequence, and high-fidelity DNA polymerase. The reaction liquid was subjected to amplification of total cDNA by 9 cycles of PCR using primers for the universal 1 and universal 2 sequences (universal oligo-long, 5'BDWTAv2). After size selection using AmPure XP beads, additional amplification was carried out by 5 cycles of PCR. The final amount of cDNA was measured by a Qubit fluorometer or nanodrop spectrophotometer (Thermo Fisher Scientific), and the size distribution of cDNA was measured by the MultiNA system (Shimadzu Corporation).

In the cDNA amplification method of the present invention, the poly(A) RNA-capture beads were subjected to reverse transcription reaction at 42°C for 30 min using reagents provided by the manufacturer and the like, and then a template-switching oligo (5'BDWTAv2-TSO) and MgCl₂were added, followed by further carrying out reverse transcription and template-switching reaction at 42°C for 30 minutes (complementary-strand synthesis step). Thereafter, exonuclease I treatment was carried out to remove unreacted poly(A) RNA-capture oligos on the beads (unreacted-oligo removal step). mRNA degradation by RNase H and homopolymer addition reaction by TdT were carried out simultaneously for 20 minutes using dCTP, and ddCTP as a terminator. After washing the beads, second-strand synthesis reaction was carried out using a primer having a guanine homopolymer at the 3'-end of the universal 2 adapter sequence, and high-fidelity DNA polymerase. The reaction liquid was subjected to amplification of total cDNA by 9 cycles of PCR using primers for the universal 1 and universal 2 sequences (universal oligo-long, 5'BDWTAv2). After size selection using AmPure XP beads (Beckman Coulter), additional amplification was carried out by 5 cycles of PCR. The final amount of cDNA was measured by a Qubit fluorometer or nanodrop spectrophotometer (Thermo Fisher Scientific), and the size distribution of cDNA was measured by the MultiNA system (Shimadzu Corporation).

The result of comparison of the cDNA yield is shown in Fig. 6. Compared to the conventional TdT method (TAS-Seq method), in which only the TdT reaction was carried out, a significantly larger amount of cDNA was obtained by the cDNA amplification method of the present invention, in which both of the TS/TdT reactions were carried out,

The result of comparison of the cDNA size distribution is shown in Fig. 7. Compared to the conventional TdT method (TAS-Seq method), in which only the TdT reaction was carried out, the cDNA amplification method of the present invention, in which both of the TS/TdT reactions were carried out, resulted in a higher peak at 1000 to 2000 base pairs (bp), suggesting that a larger amount of long full-length cDNAs were synthesized.

**[Table 1]**

| Primer name | Sequence (5'→3') | SEQ ID NO |
|---|---|---|
| universal oligo-long | NH2 (C6) /ACACTCTTTCCCTACACGACGCTCTTCCGATCT | 2 |
| 5'BDWTAv2-TSO | NH2 (C6) /AAGCAGTGGTATCAACGCAGAGTACGGG | 3 |
| 5'BDWTAv2 | NH2 (C6) /AAGCAGTGGTATCAACGCAGAG | 4 |

| | | |
|---|---|---|
| NH2(C6): 5' amino group modification, comprising a carbon spacer of six atoms. The underlined GGG of 5'BDWTAv2-TSO was designed as follows: [RNA guanine]-[RNA guanine]-[RNA guanine]. | | |

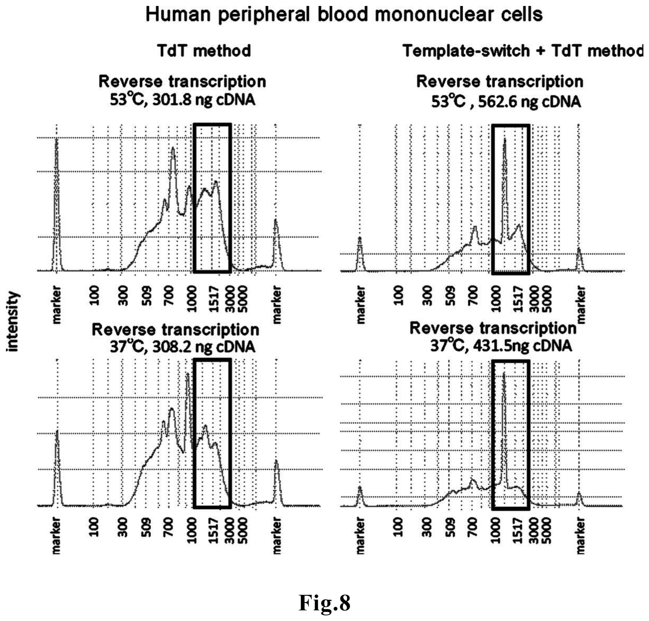

### 2. Comparison between TdT Method and cDNA Amplification Method of Present Invention in Liquid-Phase scRNA-seq Analysis System (Fig. 8)

Human PBMC specimens were obtained by the same method as in the above 1., and the cells were counted. Thereafter, the cells were loaded into a microchannel system with an appropriate density using the Chromium v3.1 reagent manufactured by 10X Genomics, according to the specifications of Chromium, to encapsulate each cell and a hydrogel bead in a microdroplet. When the cells were loaded into the Chromium, a cell lysis reagent, and a cDNA synthesis reagent comprising a reverse transcription reagent (using a reverse transcriptase having terminal transferase activity and strand displacement activity) and the like, were also loaded simultaneously. For comparison, specimens containing a template-switching oligo (10X Chromium-TSO) and specimens not containing the template-switching oligo were provided. After lysing the cells, mRNA derived from each single cell was trapped on a free poly(A) RNA-capture oligos (10X Chromium adapter) released from the hydrogel (target RNA-capturing step), and reverse transcription reaction, or reverse transcription and TS reaction, was/were carried out directly in the microdroplet at 37°C or 53°C for 1 hour (complementary-strand synthesis step). After the completion of the complementary-strand synthesis step, the emulsion constituting the microdroplet was broken by the method recommended by 10X, and cDNA was purified by nucleic acid size fractionation to remove unreacted poly(A) RNA-capture oligos and template-switching oligos (unreacted-oligo removal step). The 10X Chromium adapter is a DNA having the following structure, wherein CB is a known identification barcode consisting of 16 bases by which about 900,000 types of barcodes can be provided. In SEQ ID NO:5 of SEQUENCE LISTING, CB is represented as NNNNNNNNNNNNNNNN.

### <Structure of 10X Chromium Adapter>

CTACACGACGCTCTTCCGATCT- (CB)

NNNNNNNNNNNNTTTTTTTTTTTTTTTTTTTTTTTTTTTTTTVN (SEQ ID

NO:5)

The cDNA solution obtained was subjected to mRNA degradation by RNase H and homopolymer addition reaction by TdT simultaneously for 20 minutes using dCTP, and ddCTP as a terminator. After purification of the TdT reaction liquid, second-strand synthesis reaction was carried out using a primer having a guanine homopolymer at the 3'-end of the universal 2 adapter sequence, and high-fidelity DNA polymerase. The reaction liquid was subjected to amplification of total cDNA by 9 cycles of PCR using primers for the universal 1 and universal 2 sequences (universal oligo-long, 5'BDWTAv2). After size selection using AmPure XP beads (Beckman Coulter), the cDNA obtained was additionally amplified by 5 cycles of PCR. The final amount of cDNA was measured by a nanodrop spectrophotometer, and the size distribution of cDNA was measured by the MultiNA system.

The result of comparison of the cDNA yield and the size distribution is shown in Fig. 8. Compared to the conventional TdT method in which only the TdT reaction was carried out, the cDNA amplification method of the present invention in which both of the TS/TdT reactions were carried out resulted in production of a larger amount of cDNA and a higher peak at 1000 to 2000 base pairs (bp), suggesting that a larger amount of long full-length cDNAs were synthesized.

**[Table 2]**

| Primer name | Sequence (5'→3') | SEQ ID NO |
|---|---|---|
| universal oligo-long | NH2 (C6) /ACACTCTTTCCCTACACGACGCTCTTCCGATCT | 2 |
| 10X Chromium-TSO | AAGCAGTGGTATCAACGCAGAGTACATGGG | 6 |
| 5'BDWTAv2 | NH2 (C6) /AAGCAGTGGTATCAACGCAGAG | 4 |

| | | |
|---|---|---|
| NH2(C6): 5' amino group modification, comprising a carbon spacer of six atoms. The underlined GGG of 10X Chromium-TSO was designed as follows: [RNA guanine]-[RNA guanine]-[RNA guanine]. | | |

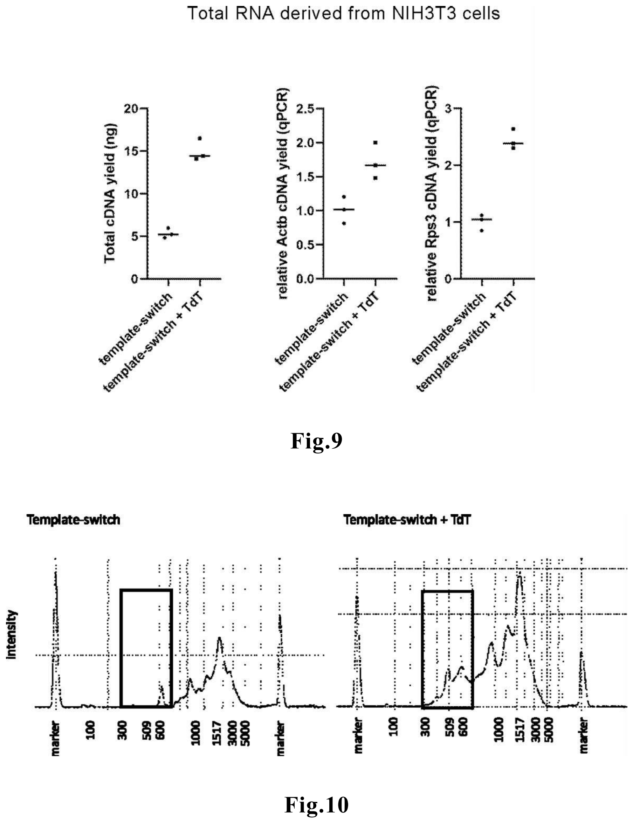

### 3. Comparison between TS Method and cDNA Amplification Method of Present Invention in Liquid-Phase cDNA Amplification System (Fig. 9, Fig. 10)

NIH3T3 cells were cultured, and total RNA was extracted using TRIzol reagent (Thermo Fisher Scientific). A poly(A) RNA-capture oligo (BioEcoP-dT25-adapter) was added to 10 ng of total RNA (six specimens) (target RNA-capturing step), and reverse transcription reaction and TS reaction were carried out using the Smart-seq2 method (Picelli S., et al. Full-length RNA-seq from single cells using Smart-seq2. Nat Protoc. 2014 Jan;9(1): 171-81.) at 42°C for 1 hour (complementary-strand synthesis step; using a reverse transcriptase having terminal transferase activity and strand displacement activity). After the completion of the complementary-strand synthesis step, cDNA was purified by nucleic acid size fractionation using Pronex beads (Promega) (unreacted-oligo removal step). Three specimens were directly subjected to amplification of total cDNA by 11 cycles of PCR using the 5'BDWTAv2 primer and the 3'WTA primer, and purified by AmPure XP. The remaining three specimens were subjected to mRNA degradation by RNase H and homopolymer addition reaction by TdT simultaneously for 20 minutes using dCTP, and ddCTP as a terminator. After purification of the TdT reaction liquid, second-strand synthesis reaction was carried out using a primer having a guanine homopolymer at the 3'-end of the universal 2 adapter sequence, and high-fidelity DNA polymerase. The reaction liquid was subjected to amplification of total cDNA by 11 cycles of PCR using primers for the universal 1 and universal 2 sequences (5'BDWTAv2 and 3'WTA primer), and purification was performed by AmPure XP. The final amount of cDNA was measured by a Qubit fluorometer; the relative contents of the *Actb* and *Rps3* genes in the cDNA were measured by quantitative real-time PCR (qPCR); and the size distribution of cDNA was measured by the MultiNA system.

The result of comparison of the cDNA yield is shown in Fig. 9. Compared to the amplification by the TS method (Smart-seq2 method) alone, the cDNA amplification method of the present invention in which both of the TS/TdT reactions were carried out resulted in a significant, about 3-fold increase in the total cDNA yield, as well as increases in the yields of the *Rps3* and *Actb* genes, suggesting a remarkable increase in cDNA synthesis efficiency.

A representative result of comparison of the cDNA distributions is shown in Fig. 10. Compared to the amplification by the TS method (Smart-seq2 method) alone, the cDNA amplification method of the present invention in which both of the TS/TdT reactions were carried out showed an increase in the ratio of cDNAs having a small size of 300 to 700 bp in the total cDNA, suggesting that the cDNA amplification method of the present invention may be able to amplify nucleic acids that hardly undergo the TS reaction, such as cDNAs generated as a result of stopping of reverse transcription in the middle of the mRNA, immature mRNAs, and partially degraded mRNAs.

**[Table 3]**

| Primer name | Sequence (5'→3') | SEQ ID NO |
|---|---|---|
| BioEcoP-dT25-adapter | 5BiotinTEG/AAGCAGTGGTATCAACGCAGAGTACTTTTTTTTTTTTTTTTTTT TTTTTT | 7 |
| Smart-seq2 TSO | biosg/AAGCAGTGGTATCAACGCAGAGTACGGG | 8 |
| 5'BDWTAv2 | NH2 (C6) /AAGCAGTGGTATCAACGCAGAG | 4 |
| 3'WTA | AAGCAGTGGTATCAACGCAGAGT | 9 |

| | | |
|---|---|---|
| 5BiotinTEG: 5' biotin-triethylene glycol modification biosg: 5' biotin modification The underlined GGG of Smart-seq2 TSO was designed as follows: [RNA guanine]-[RNA guanine]-[LNA guanine]. | | |

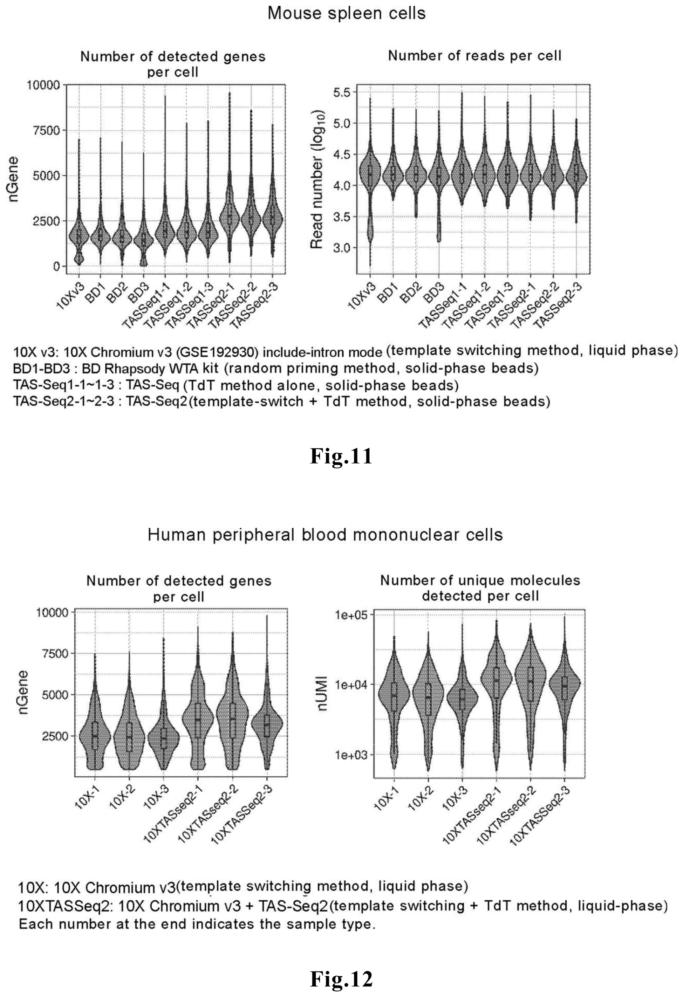

### 4. Comparison of Number of Detected Genes among TdT Method, Random Priming Method, and cDNA Amplification Method of Present Invention in scRNA-seq Analysis Using Solid-Phase Carrier (Fig. 11)

The spleen of an eight-week-old mouse was removed, and gently mashed on a 70-µm strainer placed on a 6-well plate filled with an erythrocyte hemolysis buffer (3 ml ACK buffer (155 mM NH₄Cl, 10 mM KHCO₃, 0.1 mM EDTA), followed by collecting the cell suspension. To the suspension, 7 ml of DMEM/5% FBS was added, and the resulting mixture was mixed by inversion, followed by performing centrifugation, removing the supernatant, and then washing the cells again with DMEM/5% FBS. The obtained cells were subjected to cDNA amplification using the TdT method (TAS-Seq method), the cDNA amplification method of the present invention, and the random priming provided in the BD Rhapsody kit. A reverse transcriptase having terminal transferase activity and strand displacement activity was used. After preparation of a sequencing library from the obtained cDNA, sequencing was performed using Illumina Novaseq 6000. The sequences of the primers used for the preparation of the sequencing library are shown in Table 4. The obtained sequence data were mapped on a reference sequence (GRCm38-101), to obtain gene expression data for each cell. Further, for comparison, data obtained from mouse spleen cells by 10X Chromium v3.1 were downloaded from a public database (TS method, accession ID: GSE192930). For the obtained data, down-sampling of the sequence data was carried out such that the median of the number of reads per cell should be uniform, and the data were mapped again on the reference sequence (GRCm38-101), to obtain gene expression data for each cell.

**[Table 4]**

| Primer name | Sequence (5'→3' except for Illumina adapter) | SEQ ID NO |
|---|---|---|
| Illumina adapter | 5'-Phos-GATCGGAAGAGCACACGTCTGAACTCCAGTCAC-/3AmM0/-3' | 10 |
| | 3'-T*CTAGCCTTCTCG-5' | 11 |
| ILMN_I5_UDI0065 | AATGATACGGCGACCACCGAGATCTACACATCCATATACACTCTTTCCCTACACG*A*C | 12 |
| ILMN_I5_UDI0066 | AATGATACGGCGACCACCGAGATCTACACGCTTGCGCACACTCTTTCCCTACACG*A*C | 13 |
| ILMN_I5_UDI0067 | AATGATACGGCGACCACCGAGATCTACACAGTATCTTACACTCTTTCCCTACACG*A*C | 14 |
| ILMN_I5_UDI0071 | AATGATACGGCGACCACCGAGATCTACACATTGGAACACACTCTTTCCCTACACG*A*C | 15 |
| ILMN_I5_UDI0072 | AATGATACGGCGACCACCGAGATCTACACGCCAAGGTACACTCTTTCCCTACACG*A*C | 16 |
| ILMN_I5_UDI0073 | AATGATACGGCGACCACCGAGATCTACACCGAGATATACACTCTTTCCCTACACG*A*C | 17 |
| ILMN_I5_UDI0094 | AATGATACGGCGACCACCGAGATCTACACTTCACAATACACTCTTTCCCTACACG*A*C | 18 |
| ILMN_I5_UDI0095 | AATGATACGGCGACCACCGAGATCTACACACACGAGTACACTCTTTCCCTACACG*A*C | 19 |
| ILMN_I5_UDI0096 | AATGATACGGCGACCACCGAGATCTACACGTGTAGACACACTCTTTCCCTACACG*A*C | 20 |
| ILMN_I7_UDI0065 | CAAGCAGAAGACGGCATACGAGATCTTAGTGTGTGACTGGAGTTCAGACGT*G*T | 21 |
| ILMN_I7_UDI0066 | CAAGCAGAAGACGGCATACGAGATTCCGACACGTGACTGGAGTTCAGACGT*G*T | 22 |
| ILMN_I7_UDI0067 | CAAGCAGAAGACGGCATACGAGATAACAGGAAGTGACTGGAGTTCAGACGT*G*T | 23 |
| ILMN_I7_UDI0071 | CAAGCAGAAGACGGCATACGAGATACACGAGTGTGACTGGAGTTCAGACGT*G*T | 24 |
| ILMN_I7_UDI0072 | CAAGCAGAAGACGGCATACGAGATGTGTAGACGTGACTGGAGTTCAGACGT*G*T | 25 |
| ILMN_I7_UDI0073 | CAAGCAGAAGACGGCATACGAGATGTTAATTGGTGACTGGAGTTCAGACGT*G*T | 26 |
| ILMN_I7_UDI0094 | CAAGCAGAAGACGGCATACGAGATCGCAGACGGTGACTGGAGTTCAGACGT*G*T | 27 |
| ILMN_I7_UDI0095 | CAAGCAGAAGACGGCATACGAGATGATATCGAGTGACTGGAGTTCAGACGT*G*T | 28 |
| ILMN_I7_UDI0096 | CAAGCAGAAGACGGCATACGAGATAGCGCTAGGTGACTGGAGTTCAGACGT*G*T | 29 |

| | | |
|---|---|---|
| * represents a phosphorothioate bond. Illumina adapter is an adapter prepared by annealing of two complementary primers. Phos: 5' phosphorylation; 3AmMO: 3' NH₂ modification The primers were used in the following combinations. ILMN_I5_UDI0065 and ILMN_I7_UDI0065 ILMN_I5_UDI0066 and ILMN_I7_UDI0066 ILMN_I5_UDI0067 and ILMN_I7_UDI0067 ILMN_I5_UDI0071 and ILMN_I7_UDI0071 ILMN_I5_UDI0072 and ILMN_I7_UDI0072 ILMN_I5_UDI0073 and ILMN_I7_UDI0073 ILMN_I5_UDI0094 and ILMN_I7_UDI0094 ILMN_I5_UDI0095 and ILMN_I7_UDI0095 ILMN_I5_UDI0096 and ILMN_I7_UDI0096 | | |

The result of comparison of the number of detected genes and the number of reads is shown in Fig. 11. The data obtained by the cDNA amplification method of the present invention (TAS-Seq2) were found to detect a larger number of genes compared to the other methods. By this, usefulness of the amplification method of the present invention in solid-phase single-cell transcriptome analysis was demonstrated.

### 5. Comparison of Number of Detected Genes between TS Method and cDNA Amplification Method of Present Invention in scRNA-seq Analysis Using Liquid-Phase System (Fig. 12 to Fig. 14)

Human PBMC specimens (three specimens) were obtained by the same method as in the above 1., and the cells were counted. Thereafter, the cells were loaded into a microchannel system at an appropriate density using the Chromium v3.1 reagent, manufactured by 10X Genomics, according to the specifications of Chromium, to encapsulate each cell and a hydrogel bead in a microdroplet. Two libraries were prepared per specimen. After lysing the cells, mRNA derived from each single cell was trapped on a free poly(A) RNA-capture oligo (10X Chromium adapter) released from the hydrogel (target RNA-capturing step), and reverse transcription reaction and TS reaction were carried out directly in the microdroplet at 53°C for 1 hour (complementary-strand synthesis step; using a reverse transcriptase having terminal transferase activity and strand displacement activity). After the completion of the complementary-strand synthesis step, the emulsion constituting the microdroplet was broken by the method recommended by 10X, and cDNA was purified by nucleic acid size fractionation to remove unreacted poly(A) RNA-capture oligos and template-switching oligos (unreacted-oligo removal step). One of the obtained cDNA solutions of the libraries derived from each of the three specimens was directly subjected to amplification of total cDNA by 9 cycles of PCR using primers for the universal 1 and universal 2 sequences (universal oligo-long, 5'BDWTAv2). After size selection using AmPure XP beads (Beckman Coulter), the cDNA obtained was additionally amplified by 5 cycles of PCR. For the other library derived from each of the three specimens, mRNA degradation by RNase H and homopolymer addition reaction by TdT were carried out simultaneously for 20 minutes using dCTP, and ddCTP as a terminator. After purification of the TdT reaction liquid, second-strand synthesis reaction was carried out using a primer having a guanine homopolymer at the 3'-end of the universal 2 adapter sequence, and high-fidelity DNA polymerase. The reaction liquid was subjected to amplification of total cDNA by 9 cycles of PCR using primers for the universal 1 and universal 2 sequences (universal oligo-long, 5'BDWTAv2). After size selection using AmPure XP beads (Beckman Coulter), the cDNA obtained was additionally amplified by 5 cycles of PCR.

After preparation of a sequencing library from each of the obtained cDNAs, sequencing was performed using Illumina Novaseq 6000. The sequences of the primers used for the preparation of the sequencing library are shown in Table 5. The obtained sequence data were mapped on a human reference sequence using the Cell ranger software manufactured by 10X, to obtain gene expression data for each cell. For the obtained data, down-sampling of the sequence data was carried out such that the median of the number of reads per cell should be uniform, and the data were mapped again on a human reference sequence, to obtain gene expression data for each cell.

**[Table 5]**

| Primer name | Sequence (5'→3' except for Illumina adapter) | SEQ ID NO |
|---|---|---|
| Illumina adapter | 5'-Phos-GATCGGAAGAGCACACGTCTGAACTCCAGTCAC-/AmM0/-3' | 10 |
| | 3'-T*CTAGCCTTCTCG-5' | 11 |
| ILMN_I5_UDI0020 | AATGATACGGCGACCACCGAGATCTACACGTCTCGCAACACTCTTTCCCTACACG*A*C | 30 |
| ILMN_I5_UDI0021 | AATGATACGGCGACCACCGAGATCTACACAAGACGTCACACTCTTTCCCTACACG*A*C | 31 |
| ILMN_I5_UDI0022 | AATGATACGGCGACCACCGAGATCTACACGGAGTACTACACTCTTTCCCTACACG*A*C | 32 |
| ILMN_I5_UDI0061 | AATGATACGGCGACCACCGAGATCTACACCACTACGAACACTCTTTCCCTACACG*A*C | 33 |
| ILMN_I5_UDI0062 | AATGATACGGCGACCACCGAGATCTACACTGTCGTAGACACTCTTTCCCTACACG*A*C | 34 |
| ILMN_I5_UDI0063 | AATGATACGGCGACCACCGAGATCTACACACCACTTAACACTCTTTCCCTACACG*A*C | 35 |
| ILMN_I7_UDI0020 | CAAGCAGAAGACGGCATACGAGATGTCCGTGCGTGACTGGAGTTCAGACGT*G*T | 36 |
| ILMN_I7_UDI0021 | CAAGCAGAAGACGGCATACGAGATAAGGTACCGTGACTGGAGTTCAGACGT*G*T | 37 |
| ILMN_I7_UDI0022 | CAAGCAGAAGACGGCATACGAGATGGAACGTTGTGACTGGAGTTCAGACGT*G*T | 38 |
| ILMN_I7_UDI0061 | CAAGCAGAAGACGGCATACGAGATCAGTTCCGGTGACTGGAGTTCAGACGT*G*T | 39 |
| ILMN_I7_UDI0062 | CAAGCAGAAGACGGCATACGAGATTGACCTTAGTGACTGGAGTTCAGACGT*G*T | 40 |
| ILMN_I7_UDI0063 | CAAGCAGAAGACGGCATACGAGATCTAGGCAAGTGACTGGAGTTCAGACGT*G*T | 41 |

| | | |
|---|---|---|
| * represents a phosphorothioate bond. Illumina adapter is an adapter prepared by annealing of two complementary primers. Phos: 5' phosphorylation; 3AmMO: 3' NH₂ modification The primers were used in the following combinations. ILMN_I5_UDI0061 and ILMN_I7_UDI0061 ILMN_I5_UDI0062 and ILMN_I7_UDI0062 ILMN_I5_UDI0063 and ILMN_I7_UDI0063 ILMN_I5_UDI0020 and ILMN_I7_UDI0020 ILMN_I5_UDI0021 and ILMN_I7_UDI0021 ILMN_I5_UDI0022 and ILMN_I7_UDI0022 | | |

The result of comparison of the number of detected genes and the number of reads is shown in Fig. 12. For each of the three samples from the same origin, the data obtained by the cDNA amplification method of the present invention (10XTAS-Seq2) were found to detect a larger number of genes compared to the data obtained by the TS method alone (10X).

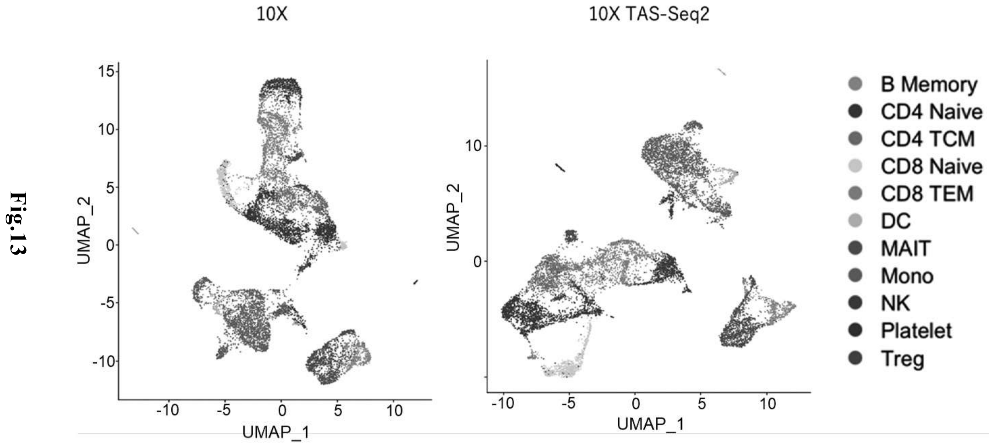

The result of cell clustering is shown in Fig. 13. Compared to the data obtained by the TS method alone (10X), the data obtained by the cDNA amplification method of the present invention (10X+TAS-Seq2) showed improved separation of various cell populations having different properties in the UMAP space.

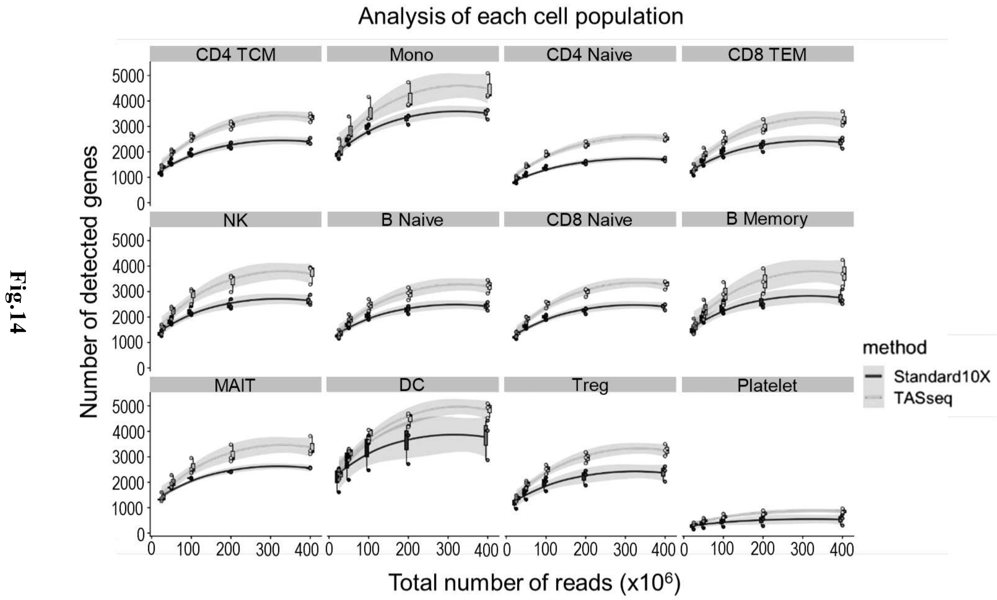

Fig. 14 shows comparison of the number of detected genes and the number of reads in various cell populations based on the result of the cell clustering. For all of the detected cell populations, the data obtained by the cDNA amplification method of the present invention (10X+TAS-Seq2) were found to detect a larger number of genes per cell compared to the data obtained by the TS method alone (10X). One of the major applications of single-cell transcriptome analysis is separation and identification of cell populations and elucidation of their properties. The present invention was thus shown to be useful in the separation and the identification of cell populations and the elucidation of their properties using liquid-phase single-cell transcriptome analysis.

Further, according to the present invention, when the same amount of analysis cost is assumed, the amount of information per cell can be increased by a factor of 1.5 to 2 compared to a conventional method. On the other hand, when about the same amount of information obtained per cell is assumed, the present invention can reduce the cost required for the sequence analysis to 1/2 to 1/3 of that of a conventional method (Fig. 14). Recently, the number of cells analyzed in scRNA-seq analysis has increased from several ten thousand to several hundred thousand. Despite such a remarkable increase in the cell throughput, the amount of sequence analysis required per cell has not changed. Therefore, the cost of the sequence analysis is expected to become the bottleneck of scRNA-seq analysis in the future. The present invention can reduce this sequencing cost, which is predicted to account for the majority of the cost of scRNA-seq analysis in the near future, to 1/2 to 1/3, thereby increasing the cost-effectiveness and making a significant contribution to the industrial application of this analysis technology.

## Claims

1. A method of amplifying a complementary DNA strand, the method comprising:
a target RNA-capturing step of capturing a target RNA with a target RNA-capture oligo comprising a target capture portion;
a complementary-strand synthesis step of carrying out reverse transcription reaction using a reverse transcriptase having terminal transferase activity, to synthesize a complementary DNA strand whose sequence is complementary to the captured RNA, and to add an additional sequence consisting of several arbitrary bases to the 3'-end of at least part of the complementary DNA molecules;
an unreacted-oligo removal step of removing a target RNA-capture oligo that has not captured the target RNA;
an RNA degradation step of degrading RNA using a ribonuclease;
a homopolymer addition step of carrying out a reaction using terminal deoxynucleotidyl transferase in the presence of dATP, dTTP, dCTP or dGTP and a chain-terminating nucleotide triphosphate, to add a nucleotide homopolymer to the 3'-end of the complementary DNA strand;
a second-strand synthesis step of synthesizing a second strand for the complementary DNA strand using a second-strand-synthesizing primer which comprises a primer comprising a complementary homopolymer portion whose sequence is complementary to the nucleotide homopolymer, to generate a DNA double strand composed of the complementary DNA strand and the second strand; and
a nucleic acid amplification step of carrying out nucleic acid amplification reaction using the DNA double strand as a template.

2. The method according to claim 1, wherein: the target RNA-capture oligo comprises a first adapter portion on the 5'-side of the target capture portion; the second-strand-synthesizing primer comprises a long primer comprising a second adapter portion on the 5'-side of the complementary homopolymer portion; and in the nucleic acid amplification step, nucleic acid amplification reaction is carried out using a primer targeting the first adapter and a primer targeting the second adapter.

3. The method according to claim 1, wherein: the second-strand-synthesizing primer comprises a long primer comprising a second adapter portion on the 5'-side of the complementary homopolymer portion; and, in the nucleic acid amplification step, nucleic acid amplification reaction is carried out using a primer targeting the second adapter and a primer targeting a desired region in the complementary DNA strand.

4. The method according to claim 2 or 3, wherein the long primer comprises, between the second adapter portion and the complementary homopolymer portion, a molecular barcode portion consisting of a random sequence.

5. The method according to claim 2 or 3, wherein the chain length of the complementary homopolymer portion of the long primer is 6 to 15 bases.

6. The method according to claim 1, wherein: the target RNA-capture oligo is a free oligo not bound to a solid-phase carrier; and the unreacted-oligo removal step is carried out by nucleic acid size fractionation.

7. The method according to claim 6, wherein the target RNA-capture oligo comprises, from the 5'-side to the 3'-side, a first adapter portion, a cell identification barcode portion, and a target capture portion.

8. The method according to claim 1, wherein: the target RNA-capture oligo is a solid-phased oligo bound to a solid-phase carrier; and the unreacted-oligo removal step is carried out by exonuclease treatment.

9. The method according to claim 8, wherein: the solid-phase carrier is a bead; and the target RNA-capture oligo comprises, from the 5'-side to the 3'-side, a first adapter portion, a bead identification barcode portion, and a target capture portion.

10. The method according to claim 8, wherein: the solid-phase carrier is a plate; and the target RNA-capture oligo is immobilized on a plurality of compartments on the plate, and comprises, from the 5'-side to the 3'-side, a first adapter portion, a compartment identification barcode portion, and a target capture portion.

11. The method according to claim 1, wherein: the target RNA-capture oligo comprises a target poly(A) RNA-capture oligo comprising a poly(T) portion as a target capture portion; and the target RNA comprises a poly(A) RNA.

12. The method according to claim 11, wherein in the complementary-strand synthesis step, reverse transcription reaction is carried out in the presence of a template-switching oligo comprising: a sequence capable of hybridizing with the additional sequence, at its 3'-end; and a switching sequence on the 5'-side of said sequence capable of hybridizing with the additional sequence; to hybridize the template-switching oligo with the complementary DNA strand having the additional sequence added thereto, and to further add a sequence complementary to the switching sequence to the 3'-end of the complementary DNA strand.

13. The method according to claim 12, wherein the template-switching oligo comprises an oligonucleotide comprising, at its 3'-end, GGG, GUG, or NGG (wherein G and U are bases of ribonucleotides, and N is a base of any ribonucleotide selected from A, U, G, and C; and wherein one or more nucleotide analogues are optionally included) as the sequence capable of hybridizing with the additional sequence, and the switching sequence comprises a second adapter portion.

14. The method according to claim 13, wherein the switching sequence comprises a molecular barcode portion consisting of a random sequence on the 3'-side of the second adapter portion.

15. The method according to claim 12, wherein the target RNA-capture oligo is a free oligo not bound to a solid-phase carrier, and wherein the unreacted-oligo removal step comprises removal of a target RNA-capture oligo that has not captured the target RNA and a template-switching oligo that has not hybridized with the complementary DNA strand by nucleic acid size fractionation.

16. The method according to claim 12, wherein the second-strand-synthesizing primer comprises: a long primer comprising a second adapter portion on the 5'-side of the complementary homopolymer portion; and a short primer comprising no complementary homopolymer portion and comprising a second adapter portion at its 3'-side region.

17. The method according to claim 16, wherein the long primer comprises, between the second adapter portion and the complementary homopolymer portion, a molecular barcode portion consisting of a random sequence.

18. The method according to claim 12, wherein in the complementary-strand synthesis step, reverse transcription reaction is carried out using a reverse transcriptase further having strand displacement activity in the presence of the template-switching oligo, to carry out second-strand synthesis for the complementary DNA strand with which the template-switching oligo has been hybridized.

19. The method according to claim 1, wherein the chain-terminating nucleotide triphosphate is ddNTP, a ddNTP derivative, 3'-dNTP, or 3'-deoxy-5-methyluridine-5'-triphosphate.

20. The method according to claim 19, wherein the ddNTP derivative is ddNTP in which the 3'-position is modified with an atomic group comprising no OH group.

21. The method according to claim 19, wherein the chain-terminating nucleotide triphosphate is ddNTP.

22. The method according to claim 1, wherein in the homopolymer addition step, dCTP and chain-terminating CTP are added to carry out poly(C) addition.

23. The method according to claim 1, wherein the RNA degradation step and the homopolymer addition step are carried out simultaneously.
